# FASCICULE DE BREVET EUROPEEN

(11) **EP 1 844 029 B1**
(45) Date de publication et mention de la délivrance du brevet: **12.11.2008**
(21) Numéro de dépôt: 06709172.8
(22) Date de dépôt: 26.01.2006
(51) Int. Cl.: C07D 279/16, C07D 265/36, A61K 31/538, A61K 31/5415, A61P 3/06, A61P 3/10

(54) **NOUVEAUX DERIVES D'OXIMES HETEROCYCLIQUES, LEUR PROCEDE DE PREPARATION ET LEUR UTILISATION COMME AGENTS HYPOGLYCEMIANTS ET HYPOLIPEMIANTS**
NEUE HETEROCYCLISCHE OXIMDERIVATE, HERSTELLUNGSVERFAHREN DAFÜR UND IHRE VERWENDUNG ALS HYPOGLYKÄMISCHE ODER HYPOLIPIDEMISCHE WIRKSTOFFE
NOVEL HETEROCYCLIC OXIME DERIVATIVES, METHOD FOR PREPARING SAME AND USE THEREOF AS HYPOGLYCAEMIC OR HYPOLIPIDEMIC AGENTS

(30) Priorité: 27.01.2005 FR 0500841
(43) Date de publication de la demande: 17.10.2007
(73) Titulaire: Les Laboratoires Servier, 92415 Courbevoie Cedex (FR)
(72) Inventeur: HURTEVENT, Aurélie, F-59000 Lille (FR); L'HELGOUAL'CH, Jean-Martial, F-59000 Lille (FR); CARATO, Pascal, F-59000 Lille (FR); LEBEGUE, Nicolas, F-59480 La Bassee (FR); LECLERC, Véronique, F-59710 Avelin (FR); BERTHELOT, Pascal, F-59320 Haubourdin (FR); DACQUET, Catherine, 59650 Villeneuve d'Ascq (FR); KTORZA, Alain, F-94130 Nogent-sur-Marne (FR); CAIGNARD, Daniel-Henri, F-78230 Le Pecq (FR)
(86) Numéro de dépôt international: PCT/FR2006/000175
(87) Numéro de publication internationale: WO 2006/079720

(56) Documents cités:
- EP-A- 1 466 604

## Description

La présente invention concerne de nouveaux dérivés d'oximes hétérocycliques, leur procédé de préparation et les compositions pharmaceutiques qui les contiennent.

Les composés décrits dans la présente invention sont nouveaux et présentent des propriétés pharmacologiques particulièrement intéressantes : ce sont d'excellents agents hypoglycémiants et hypolipémiants.

Le traitement du diabète non insulino-dépendant de type II reste non satisfaisant en dépit de la mise sur le marché de nombreux dérivés hypoglycémiants oraux destinés à faciliter la sécrétion d'insuline et à favoriser son action au niveau des tissus cibles périphériques.

Lors des dix dernières années, une classe de composés de structure thiazolidinediones (US 5089514, US 5306726) a montré une activité antidiabétique marquée en favorisant la sensibilité à l'insuline dans les tissus périphériques cibles (muscles squelettiques, foie, tissu adipeux) de modèles animaux de diabète non insulino-dépendant de type II. Ces composés réduisent également les taux d'insuline et de lipides dans ces mêmes modèles animaux et induisent *in vitro* la différenciation de lignées cellulaires de préadipocytes en adipocytes (A. Hiragun et al., J. Cell. Physiol., 1988, 134, 124-130 ; R.F. Kleitzen et al., Mol. Pharmacol., 1992, 41, 393-398).
Le traitement des lignées cellulaires préadipocytaires avec la thiazolidinedione Rosiglitazone entraîne une induction de l'expression de gènes spécifiques du métabolisme lipidique comme aP2 et l'adipsine ainsi que l'expression des transporteurs du glucose GLUT1 et GLUT4, suggérant que l'effet des thiazolidinediones observé *in vivo* peut-être médié via le tissu adipeux. Cet effet spécifique est obtenu par la stimulation des facteurs nucléaires de transcription : « peroxisome proliferator-activated receptor gamma » (PPAR γ2). Ces dérivés sont susceptibles de restaurer la sensibilité à l'insuline au niveau des tissus périphériques tels que le tissu adipeux ou les muscles squelettiques (J.E. Gerich, New Engl. Med., 19, 321, 1231-1245).
Néanmoins, les dérivés de structure thiazolidinediones (troglitazone, rosiglitazone) ont montré chez l'homme des effets secondaires inquiétants, notamment des problèmes hépatiques (Script N° 2470, 1999, Sept. 8th, 25).
De nombreux agents hypoglycémiants présentent des effets secondaires importants (hépatiques, cardiaques, hématopoïétiques) qui limitent leur utilisation à long terme dans le traitement du diabète non insulino-dépendant de type II.
Le développement de nouveaux agents thérapeutiques moins toxiques et actifs à long terme est absolument nécessaire dans cette pathologie.
Par ailleurs, l'hyperlipidémie est souvent observée chez les diabétiques (Diabete Care, 1995, 18 (supplément 1), 86/8/93). L'association de l'hyperglycémie avec l'hyperlipidémie favorise le risque de maladies cardiovasculaires chez les diabétiques. L'hyperglycémie, l'hyperlipidémie et l'obésité sont devenues des pathologies du monde moderne marqué par une prise de nourriture en grande quantité et un manque chronique d'exercice.
L'augmentation de la fréquence de ces pathologies appelle au développement de nouveaux agents thérapeutiques actifs dans ces maladies : des composés présentant une excellente activité hypoglycémiante et hypolipémiante en évitant les effets secondaires observés avec les thiazolidinediones sont par conséquent très utiles dans le traitement et/ou la prophylaxie de ces pathologies et particulièrement indiqués dans le traitement des diabètes non insulino-dépendants de type II pour réduire l'insulino-résistance périphérique et normaliser le contrôle du glucose.

Les composés de la présente invention, outre leur nouveauté, répondent à ces critères pharmacologiques et constituent d'excellents agents hypoglycémiants et hypolipémiants.

La présente invention concerne plus particulièrement les composés de formule (I) : 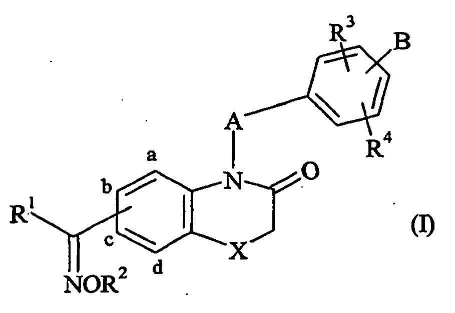 dans laquelle :
- X représente un atome d'oxygène ou de soufre,
- A représente une chaîne alkylène (C₁-C₆) dont un groupement CH₂ peut être optionnellement remplacé par un hétéroatome choisi parmi oxygène ou soufre, ou par un groupement NRₐ (où Rₐ représente un atome d'hydrogène ou un groupement alkyle (C₁-C₆) linéaire ou ramifié), ou par un groupement phénylène ou naphtylène,
- R¹ et R², identiques ou différents, représentent un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié,
- R³ et R⁴, identiques ou différents, représentent un atome d'hydrogène ou d'halogène, ou un groupement R, OR ou NRR', où R et R', identiques ou différents, représentent un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié,
ou R³ et R⁴ forment ensemble avec les atomes de carbone qui les portent, lorsqu'ils sont portés par deux atomes de carbone adjacents, un cycle comportant 5 ou 6 chaînons et pouvant optionnellement contenir un hétéroatome choisi parmi oxygène, soufre et azote,
- B représente un groupement alkyle (C₁-C₆) linéaire ou ramifié, ou alkényle (C₂-C₆) linéaire ou ramifié, ces groupements étant substitués :
   ◆ par un groupement de formule (II): 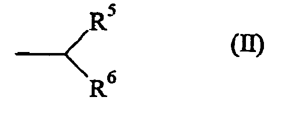 dans laquelle :
      * R⁵ représente un groupement 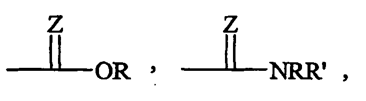 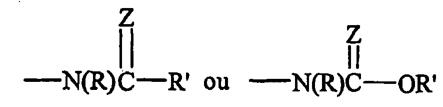
      dans lesquels Z représente un atome d'oxygène ou de soufre et R et R', identiques ou différents, sont tels que définis précédemment,
      * et R⁶ représente un groupement aryle, arylalkyle dont la partie alkyle contient de 1 à 6 atomes de carbone et peut être linéaire ou ramifiée, hétéroaryle, hétéroarylalkyle dont la partie alkyle contient 1 à 6 atomes de carbone et peut être linéaire ou ramifié,
         CN, Tétrazole, -OR , -NRR' , 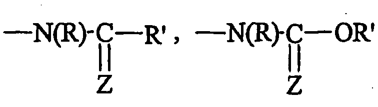
      ou 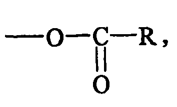 dans lesquels Z est tel que défini précédemment et R et R', identiques ou différents, peuvent prendre les mêmes valeurs que défini précédemment,
   ◆ ou par un groupement R⁷ où R⁷ représente un groupement CN, tétrazole, 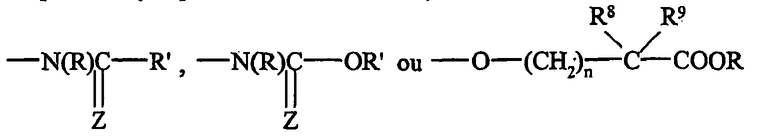
   dans lesquels Z est tel que défini précédemment et R et R', identiques ou différents, peuvent prendre les mêmes valeurs que défini précédemment, n représente 0, 1, 2, 3, 4, 5 ou 6, et R⁸ et R⁹, identiques ou différents, représentent un atome d'hydrogène ou un groupement alkyle (C₁-C₆) linéaire ou ramifié étant entendu que R⁸ et R⁹ ne peuvent représenter simultanément un atome d'hydrogène,
ou B représente un groupement de formule (II) ou un groupement R⁷ tels que définis précédemment,
étant entendu que :
* l'oxime R¹-C(=N-OR²)- peut être de configuration Z ou E,
* par aryle on entend un groupement phényle, naphtyle ou biphényle, ces groupements pouvant être optionnellement partiellement hydrogénés,
* par hétéroaryle on entend tout groupement aromatique mono ou bicyclique contenant 5 à 10 chaînons, pouvant être optionnellement partiellement hydrogéné sur un des cycles dans le cas des hétéroaryles bicycliques, et contenant 1 à 3 hétéroatomes choisis parmi oxygène, azote et soufre,
les groupements aryle et hétéroaryle ainsi définis pouvant être optionnellement substitués par 1 à 3 groupements, identiques ou différents, choisis parmi alkyle (C₁-C₆) linéaire ou ramifié, polyhalogénoalkyle (C₁-C₆) linéaire ou ramifié, alkoxy (C₁-C₆) linéaire ou ramifié, hydroxy, carboxy, formyle, acyle (C₁-C₆) linéaire ou ramifié, aroyle, NR_{b}R_{c} (dans lequel R_{b} et R_{c}, identiques ou différents, représentent un atome d'hydrogène, un groupement alkyle (C₁-C₆) linéaire ou ramifié, aryle ou hétéroaryle), ester, amido, nitro, cyano, ou atomes d'halogène,
leurs énantiomères et diastéréoisomères ainsi que leurs sels d'addition à un acide ou à une base pharmaceutiquement acceptables.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon préférée, le groupement R¹-C(=N-OR²)- est en position b ou c.

Le groupement préféré pour R³ et R⁴ est l'atome d'hydrogène.

Préférentiellement, A représente une chaîne alkylène dont un groupement CH₂ peut être optionnellement remplacé par un hétéroatome et plus particulièrement par un atome d'oxygène.
Plus particulièrement, l'invention concerne les composés de formule (I) pour lesquels A représente un groupement éthylèneoxy.

Les groupements R² préférés sont l'atome d'hydrogène et le groupement alkyle, comme par exemple le groupement méthyle.

R¹ représente avantageusement un groupement phényle non substitué ou substitué par un ou plusieurs substituants choisis parmi des groupements tels que alkyle, alkoxy, et atomes d'halogène, et plus particulièrement R¹ représente un groupement phényle non substitué.

Les groupements B préférés sont les groupements alkyle ou alkényle, et plus particulièrement les groupements alkyle, substitués par un groupement 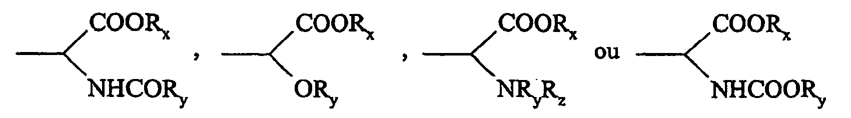 dans lesquels Rₓ, R_{y} et R_{z}, identiques ou différents, représentent :
un atome d'hydrogène ou un groupement alkyle comme par exemple les groupements méthyle, éthyle, propyle, isopropyle, butyle, isobutyle, tertbutyle, pentyle, isopentyle, néopentyle, hexyle,
un groupement polyhalogéno alkyle comme par exemple les groupements trifluorométhyle ou trifluoroéthyle,
   ou un groupement phényle ou benzyle.

Plus particulièrement, l'invention concerne les composés de formule (I) pour lesquels B représente un groupement alkyle ou alkényle substitué par un groupement 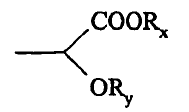 où Rₓ, et R_{y} sont tels que défninis précédemment.

B représente également avantageusement un groupement 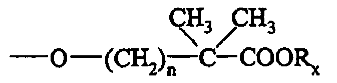 dans lequel n et Rₓ sont tels que définis précédemment.

Encore plus préférentiellement, B représente un groupement 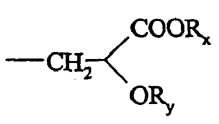 dans lequel Rₓ et R_{y} sont tels que définis précédemment.

Très avantageusement, l'invention concerne les composés de formule (I) pour lesquels :
X représente un atome d'oxygène ou de soufre,
A représente une chaîne 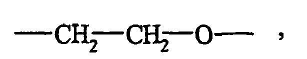 R³ et R⁴ représentent simultanément un atome d'hydrogène,
R² représente un atome d'hydrogène ou un groupement alkyle,
R¹ représente un groupement phényle non substitué,
B représente un groupement 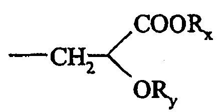 dans lequel Rₓ et R_{y} sont tels que définis précédemment.

Encore plus particulièrement, l'invention concerne les composés de formule (I) qui sont :
* le 2-éthoxy-3-[4-(2-{7-[(méthoxyimino)(phényl)méthyl]-3-oxo-2,3-dihydro-4*H*-1,4-benzothiazin-4-yl}éthoxy)phényl]propanoate d'éthyle,
* 3-[4-(2-{7-[(méthoxyimino)(phényl)méthyl]-3-oxo-2,3-dihydro-4*H*-1,4-benzothiazin-4-yl}éthoxy)phényl]-2-(2,2,2-trifluoroéthoxy)propanoate d'éthyle,
* 2-éthoxy-3-[4-(2-{6-[(méthoxyimino)(phényl)méthyl]-3-oxo-2,3-dihydro-4*H*-1,4-benzoxazin-4-yl}éthoxy)phényl]propanoate d'éthyle,
* le 2-éthoxy-3-[4-(2-{7-[(hydroxyimino)(phényl)méthyl]-3-oxo-2,3-dihydro-4*H*-1,4-benzothiazin-4-yl}éthoxy)phényl]propanoate d'éthyle,
* 3-[4-(2-{7-[(hydroxyimino)(phényl)méthyl]-3-oxo-2,3-dihydro-4*H*-1,4-benzothiazin-4-yl}éthoxy)phényl]-2-(2,2,2-trifluoroéthoxy)propanoate d'éthyle
* 2-éthoxy-3-[4-(2-{6-[(hydroxyimino)(phényl)méthyl]-3-oxo-2,3-dihydro-4*H*-1,4-benzoxazin-4-yl}éthoxy)phényl]propanoate d'éthyle,
* l'acide 2-éthoxy-3-[4-(2-{7-[(hydroxyimino)(phényl)méthyl]-3-oxo-2,3-dihydro-4*H-*1,4-benzothiazin-4-yl}éthoxy)phényl]propanoïque,
* l'acide 2-éthoxy-3-[4-(2-{7-[(méthoxyimino)(phényl)méthyl]-3-oxo-2,3-dihydro-4*H-*1,4-benzothiazin-4-yl} éthoxy)phényl]propanoïque,
* l'acide 3-[4-(2-{7-[(hydroxyimino)(phényl)méthyl]-3-oxo-2,3-dihydro-4*H-*1,4-benzothiazin-4-yl}éthoxy)phényl]-2-(2,2,2-trifluoroéthoxy)propanoïque,
* l'acide 3-[4-(2-{7-[(méthoxyimino)(phényl)méthyl]-3-oxo-2,3-dihydro-4*H*-1,4-benzothiazin-4-yl}éthoxy)phényl]-2-(2,2,2-trifluoroéthoxy)propanoïque,
* l'acide 2-éthoxy-3-[4-(2-{6-[(hydroxyimino)(phényl)méthyl]-3-oxo-2,3-dihydro-4*H-*1,4-benzoxazin-4-yl}éthoxy)phényl]propanoïque,
* l'acide 2-éthoxy-3-[4-(2-{6-[(méthoxyimino)(phényl)méthyl]-3-oxo-2,3-dihydro-4*H-*1,4-benzoxazin-4-yl}éthoxy)phényl]propanoïque.

Les énantiomères, diastéréoisomères ainsi que les sels d'addition à un acide ou à une base pharmaceutiquement acceptables des composés préférés de l'invention font partie intégrante de l'invention.

La présente invention concerne également le procédé de préparation des composés de formule (I) **caractérisé en ce que** l'on utilise comme produit de départ un composé de formule (III) : 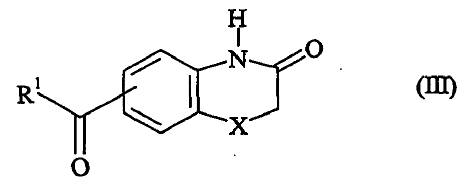 dans laquelle R¹ et X sont tels que définis dans la formule (I),
sur lequel on condense en milieu basique un composé de formule (IV) : 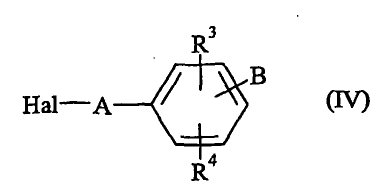 dans laquelle A, B, R³ et R⁴ sont tels que définis dans la formule (I) et Hal représente un atome d'halogène, pour conduire au composé de formule (V) : 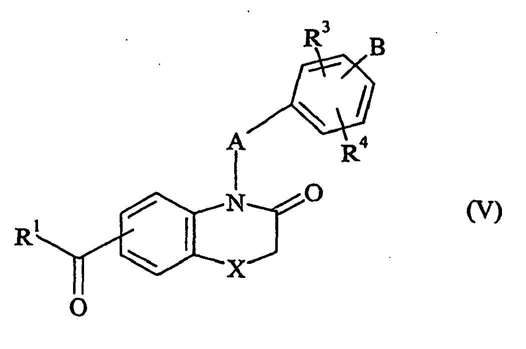 dans laquelle R¹, R³, R⁴, A, B et X sont tels que définis dans la formule (I),
que l'on soumet à l'action d'un composé de formule R²O-NH₂ dans laquelle R² est tel que défini dans la formule (I) pour conduire au composé de formule (I) : 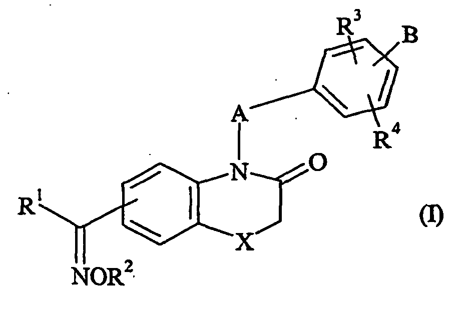 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

Une variante avantageuse concerne le procédé de préparation des composés de formule (I) caractérisé en ce que l'on utilise comme produit de départ un composé de formule (III) : 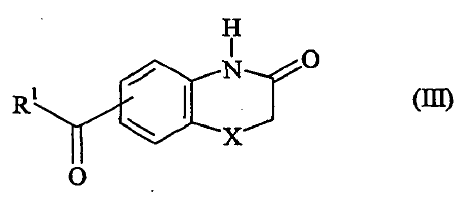 dans laquelle R¹ et X sont tels que définis dans la formule (I),
sur lequel on condense un composé de formule R²O-NH2 dans laquelle R² est tel que défini dans la formule (I) pour conduire au composé de formule (VI) : 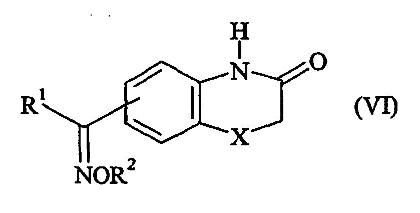 dans laquelle R¹, R² et X sont tels que définis dans la formule (I),
sur lequel on condense en milieu basique un composé de formule (IV) : 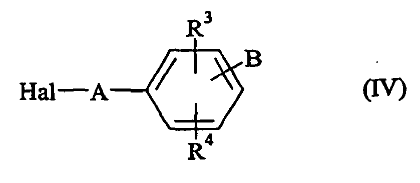 dans laquelle A, B, R³ et R⁴ sont tels que définis dans la formule (I) et Hal représente un atome d'halogène, pour conduire au composé de formule (I) : 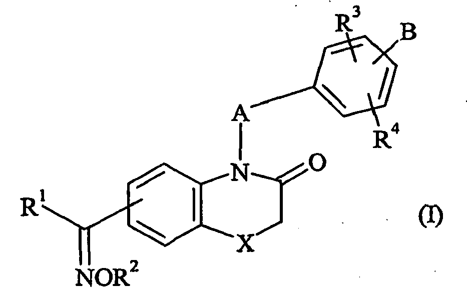 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.
Les composés de formule (III) sont commerciaux, ou aisément accessibles à l'homme du métier par des réactions de chimie classiques, ou décrits dans la littérature.

Les composés de la présente invention possèdent des propriétés pharmacologiques très intéressantes.

Ces composés montrent notamment une excellente activité dans la réduction des taux de glucose sanguin. Ces propriétés justifient leur application en thérapeutique dans le traitement et/ou la prophylaxie des hyperglycémies, des dyslipidémies et plus particulièrement dans le traitement des diabètes non insulino-dépendants de type II, l'intolérance au glucose, les désordres reliés au syndrome X (incluant l'hypertension, l'obésité, la résistance à l'insuline, l'athérosclérose, l'hyperlipidémie), les maladies artérielles coronaires et d'autres maladies cardiovasculaires (incluant l'hypertension artérielle, l'insuffisance cardiaque, l'insuffisance veineuse), les maladies rénales (incluant les glomérulonéphrites, les gloméruloscléroses, le syndrôme néphrotique, la néphrosclérose hypertensive), les rétinopathies, les désordres reliés à l'activation des cellules endothéliales, le psoriasis, le syndrôme polycystique ovarien, la démence, les complications diabétiques et l'ostéoporose.
Ils peuvent être utilisés comme inhibiteurs de l'aldose réductase, pour améliorer les fonctions cognitives dans la démence et les complications du diabète, les maladies inflammatoires intestinales, les dystrophies myotoniques, les pancréatites, l'artériosclérose, le xanthome.

L'activité de ces composés est également recommandée pour le traitement et/ou la prophylaxie d'autres maladies incluant le diabète de type I, les hypertriglycéridémies, le syndrome X, la résistance à l'insuline, les dyslipidémies chez le diabétique, les hyperlipidémies, l'hypercholestérolémie, l'hypertension artérielle, l'insuffisance cardiaque, les maladies cardiovasculaires notamment l'athérosclérose.

De plus, ces composés sont indiqués pour être utilisés dans la régulation de l'appétit, notamment dans la régulation de la prise de nourriture chez des sujets souffrant de désordres tels que l'obésité, l'anorexie, la boulimie et l'anorexie nerveuse.
Ainsi, ces composés peuvent être utilisés en prévention ou pour le traitement de l'hypercholestérolémie, de l'obésité avec des effets avantageux sur l'hyperlipidémie, l'hyperglycémie, l'ostéoporose, l'intolérance au glucose, la résistance à l'insuline ou les maladies dans lesquelles l'insulino-résistance est un mécanisme physiopathologique secondaire.

L'utilisation de ces composés permet de réduire le cholestérol total, le poids corporel, la résistance à la leptine, le glucose plasmatique, les triglycérides, les LDL, les VLDL ainsi que les acides gras libres plasmatiques. Ils peuvent être utilisés en association avec des inhibiteurs de la HMG CoA réductase, les fibrates, l'acide nicotinique, la cholestyramine, le colestipol, le probucol, le GLP1, la metformine, les biguanides ou les inhibiteurs de la réabsorption du glucose, et peuvent être administrés ensembles ou à des périodes différentes pour agir en synergie chez le patient traité.

Ils présentent par ailleurs une activité dans les pathologies cancéreuses et notamment les cancers hormono-dépendants tels le cancer du sein et le cancer du colon, ainsi qu'un effet inhibiteur des processus d'angiogénèse impliqués dans ces pathologies.

Parmi les compositions pharmaceutiques selon l'invention, on pourra citer plus particulièrement celles qui conviennent pour l'administration orale, parentérale, nasale, per
ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou plusieurs prises.

La présente invention concerne également une nouvelle association entre un dérivé hétérocyclique de formule (I) tel que défini précédemment et un agent antioxydant pour l'obtention de compositions pharmaceutiques utiles dans le traitement et/ou la prévention de l'obésité et des surcharges pondérales caractérisées par un index de poids corporel supérieur à 25.
Les agents antioxydants selon l'invention sont plus particulièrement des agents antiradicalaires ou piégeurs de radicaux libres, des agents antilipopéroxydants, des agents chélatants ou des agents capables de régénérer les antioxydants endogènes comme le glutathion, la vitamine C ou la vitamine E, ainsi que leurs sels d'addition à un acide ou à une base pharmaceutiquement acceptable.

L'agent antioxydant de l'association selon l'invention est plus préférentiellement représenté par des dérivés de quinones comme l'ubiquinone ou coenzyme Q₁₀, qui agit en tant que piégeur de radicaux libres mais qui est également capable de régénérer de la vitamine E.

L'association préférée selon l'invention est l'acide 3-{4-[2-(6-(hydroxyimino-phényl)-méthyl-3-oxo-2,3-dihydro-4H-benzo[1,4]oxazin-4-yl)-éthoxy]-phényl}-2-éthoxy-propanoïque et le coenzyme Q₁₀.

Par ailleurs, l'association selon l'invention possède des propriétés pharmacologiques tout à fait surprenantes : la demanderesse a en effet mis en évidence l'existence d'une synergie entre les deux composés de l'association permettant d'obtenir une réduction très significative de la masse grasse corporelle la rendant utile dans le traitement et/ou la prévention de l'obésité et des surcharges pondérales caractérisées par un index de poids corporel supérieur à 25.

L'obésité aux Etats-Unis atteint 20 % des hommes et 25 % des femmes. Sont considérés comme obèses les patients d'indice de poids corporel (IMC = poids (kg) / taille² (m²)) supérieur ou égal à 30 (Int. J. Obes., 1998, 22, 39-47 ; Obesity Lancet, 1997, 350, 423-426). L'obésité (IMC ≥ 30) et les surcharges pondérales (25 < IMC < 30) peuvent avoir plusieurs origines : elles peuvent survenir à la suite d'une dérégulation de la prise de nourriture, d'une dérégulation hormonale ou encore à la suite de l'administration d'un traitement : un traitement antidiabétique de type II avec les sulfonylurées entraîne une prise de poids chez les patients. De même dans le diabète de type I (insulino-dépendant), l'insulinothérapie est également une source de prise de poids corporel chez les malades (In Progress in Obesity Research, 8th International Congress on Obesity, 1999, 739-746 ; Annals of Internal Medicine, 1998, 128, 165-175).

L'obésité et les surcharges pondérales sont des facteurs de risque bien établis pour les maladies cardiovasculaires : elles sont associées à une augmentation signification des risques d'accidents vasculaires cérébraux, de diabète non-insulino-dépendant car elles prédisposent à l'insulino-résistance, aux dyslipidémies et à l'apparition de maladies macrovasculaires (néphropathies, rétinopathies, angiopathies).
D'autres pathologies sont la conséquence de l'obésité ou de surcharges pondérales : on peut citer en particulier les calculs vésiculaires, les dysfonctions respiratoires, plusieurs formes de cancers et dans les cas d'obésité très sévère la mort prématurée (N. Engl. J. Med., 1995, 333, 677-385 ; JAMA, 1993, 270, 2207-2212).
L'association selon l'invention permet d'obtenir une perte de poids qui même modérée réduit significativement tous les facteurs de risque associés à l'obésité (Int. J. Obes., 1997, 21, 55-9 ; Int. J. Obes., 1992, 21, S5-9).
L'association selon l'invention trouve donc son utilité dans le traitement et/ou la prévention de l'obésité et des surcharges pondérales caractérisées par un index corporel supérieur à 25.
L'invention concerne donc l'utilisation de l'association entre un composé de formule (I) et un agent antioxydant pour l'obtention de compositions pharmaceutiques destinées au traitement et/ou à la prévention de l'obésité et des surcharges pondérales caractérisées par un index corporel supérieur à 25 et inférieur à 30.

En particulier, l'association selon l'invention est utile dans le traitement et/ou la prévention de l'obésité et des surcharges pondérales caractérisées par un index corporel supérieur à 25 et inférieur à 30 induites par un traitement thérapeutique, comme le traitement du diabète de type I ou II.
L'invention concerne donc l'utilisation de l'association entre un composé de formule (I) et un agent antioxydant pour l'obtention de compositions pharmaceutiques destinées au traitement et/ou à la prévention de l'obésité et des surcharges pondérales caractérisées par un index corporel supérieur à 25 et inférieur à 30 induites par un traitement thérapeutique, comme le traitement du diabète de type I ou II.

L'invention concerne également les compositions pharmaceutiques contenant l'association entre un composé de formule (I) et un agent antioxydant telle que définie précédemment en combinaison avec un ou plusieurs excipients pharmaceutiquement acceptable.
Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

En particulier, l'invention concerne les compositions pharmaceutiques contenant un composé de formule (I) telle que définie précédemment et un agent antioxydant comme le coenzyme Q₁₀ ou la vitamine E en combinaison avec un ou plusieurs excipients pharmaceutiquement acceptables.

La posologie varie selon le sexe, l'âge et le poids du patient, la voie d'administration, la nature de l'indication thérapeutique ou des traitements éventuellement associés et s'échelonne entre 0,1 mg et 1 g de chaque composant de l'association par 24 heures en une ou plusieurs prises.

Les préparations et exemples suivants illustrent l'invention et ne la limitent en aucune façon.

### Préparation 1 : 7-{[Méthoxyimino]-phényl-méthyl}-2H,4H-benzo[1,4]thiazin-3-one

### Stade A : 7-Benzoyl-2H,4H-benzo[1,4]thiazin-3-one

Dans 100 g d'acide polyphosphorique, on ajoute 7,4 g d'acide benzoïque puis 10 g de 4*H-*benzo[1,4]thiazin-3-one. Le milieu est porté à 160°C sous agitation mécanique pendant 6 heures.

La solution est ensuite hydrolysée, le précipité essoré et lavé avec une solution d'hydrogénocarbonate de sodium saturée puis avec de l'alcool absolu. Le solide obtenu est recristallisé dans le toluène pour conduire au produit du titre sous la forme d'une poudre beige.
*Point de fusion : 211-213°C*

### Stade B : 7-{[Méthoxyimino]-phényl-méthyl}-2H,4H-benzo[1,4]thiazin-3-one

Dans 40 ml de méthanol, on dissoud 5 g du composé obtenu au Stade A puis 3 g de chlorhydrate de méthoxylamine et 5,7 g de pyridine. Le milieu est porté à reflux sous agitation pendant 4 heures. Après l'arrêt du chauffage, on laisse précipiter le produit. Le précipité est filtré et correspond à , l'isomère « E » du composé du titre. On hydrolyse et acidifie le filtrat avec de l'HCl 3N puis on filtre le précipité, qui correspond à l'isomère « Z » du composé du titre.
*Point de fusion : 214-216°C (isomère « E* »)
*138-140°C (mélange « E » et « Z » : 72*/*28%)*

### Préparation 2 : 6-{[Méthoxyimino]-phényl-méthyl}-2H,4H-benzo[1,4]oxazin-3-one

### Stade A : 6-Benzoyl-2H,4H-benzo[1,4]oxazin-3-one

Dans 100 g d'acide polyphosphorique, on ajoute 8,2 g d'acide benzoïque puis 10 g de 2*H-*[1,4]-benzoxazin-3-one. Le milieu est porté à 160°C sous agitation mécanique pendant 6 heures.

La solution est ensuite hydrolysée, le précipité essoré et lavé avec une solution d'hydrogénocarbonate de sodium saturée puis avec de l'alcool absolu. Le solide obtenu est recristallisé dans le toluène pour conduire au produit du titre sous la forme d'une poudre blanche.
*Point de fusion : 154-156°C*

### Stade B : 6-{[Méthoxyimino]-phényl-méthyl}-2H,4H-benzo[1,4]oxazin-3-one

Dans 40 ml de méthanol, on dissoud 5 g du composé obtenu au Stade A puis 3 g de chlorhydrate de méthoxylamine et 5,7 g de pyridine. Le milieu est porté à reflux sous agitation pendant 4 heures. Après l'arrêt du chauffage, on hydrolyse et acidifie le filtrat avec de l'HCl 3N puis on filtre le précipité, qui correspond au composé du titre.
*Point de fusion : 147-149°C*

### Préparation 3 : 3-{4-[2-(7-Benzoyl-3-oxo-2,3-dihydro-4H-benzo[1,4]thiazin-4-yl)-éthoxy]-phényl}-2-éthoxy-propanoate d'éthyle

Dans 30 ml de diméthylformamide, on dissoud 1 g du composé obtenu au Stade A de la Préparation 1 puis on ajoute 1 g carbonate de potassium, et on laisse sous agitation à 80°C pendant une heure. Le 3-[4-(2-chloroéthoxy)-phényl]-2-éthoxy-propanoate d'éthyle (1,67 g) est dissout dans un minimum de diméthylformamide et on laisse sous agitation à 110°C pendant une nuit. Le diméthylformamide est ensuite évaporé, puis la solution est hydrolysée et acidifiée. La phase aqueuse est extraite avec du dichlorométhane, séchée sur sulfate de magnésium, filtrée et la phase organique évaporée. Le résidu est purifié sur gel de silice avec du toluène et de l'acétate d'éthyle (8/2) comme éluant pour conduire au produit du titre sous la forme d'une huile.

### Préparation 4 : 3-{4-[2-(7-Benzoyl-3-oxo-2,3-dihydro-4H-benzo[1,4]thiazin-4-yl)-éthoxy]-phényl}-2-(2,2,2-trifluoroéthoxy)-propanoate d'éthyle

Dans 30 ml de diméthylformamide, on dissout 1 g du composé obtenu au Stade A de la Préparation 1 puis on ajoute 1g carbonate de potassium, et on laisse sous agitation à 80°C pendant une heure. Le 3-[4-(2-chloroéthoxy)-phényl]-2-(2,2,2-trifluoroéthoxy) propanoate d'éthyle (1,97 g) est dissout dans un minimum de diméthylformamide et on laisse sous agitation à 110°C pendant une nuit. Le diméthylformamide est ensuite évaporé, puis la solution est hydrolysée et acidifiée. La phase aqueuse est extraite avec du dichlorométhane, séchée sur sulfate de magnésium, filtrée et la phase organique évaporée. Le résidu est purifié sur gel de silice avec du toluène et de l'acétate d'éthyle (8/2) comme éluant pour conduire au produit du titre sous la forme d'une huile.

### Préparation 5 : 3-{4-[2-(6-Benzoyl-3-oxo-2,3-dihydro-4H-benzo[1,4]oxazin-4-yl)-éthoxy]-phényl}-2-éthoxy-propanoate d'éthyle

Dans 30 ml de diméthylformamide, on dissoud 1 g du composé obtenu dans le Stade A de la Préparation 2 puis on ajoute 1,1 g carbonate de potassium, et on laisse sous agitation à 80°C pendant une heure. Le 3-[4-(2-chloroéthoxy)-phényl]-2-éthoxy-propanoate d'éthyle (1,78 g) est dissout dans un minimum de diméthylformamide et on laisse sous agitation à 110°C pendant une nuit.
Le diméthylformamide est ensuite évaporé, puis la solution est hydrolysée et acidifiée. La phase aqueuse est extraite avec du dichlorométhane, séchée sur sulfate de magnésium, filtrée et la phase organique évaporée. Le résidu est purifié sur gel de silice avec du toluène et de l'acétate d'éthyle (8/2) comme éluant pour conduire au produit du titre sous la forme d'une huile.

### Préparation 6 : 6-{[Méthoxyimino]-phényl-méthyl}-2H,4H-benzo[1,4]thiazin-3-one

### Stade A : 6-Benzoyl-2H,4H-benzo[1,4]thiazin-3-one

### Stade B : 6-{[Méthoxyimino]-phényl-méthyl}-2H,4H-benzo[1,4]thiazin-3-one

On procède comme dans la Préparation 2 en remplaçant la 2*H*-[1,4]-benzoxazin-3-one par la 2*H-*[1,4]-benzothiazin-3-one.

### Préparation 7 : 7-{[Méthoxyimino]-phényl-méthyl}-2H,4H-benzo[1,4]oxazin-3-one

### Stade A : 7-Benzoyl-2H,4H-benzo[1,4]oxazin-3-one

### Stade B : 7-{[Méthoxyimino]-phényl-méthyl}-2H,4H-benzo[1,4]oxazin-3-one

On procède comme dans la Préparation 1 en remplaçant la 4*H*-benzo[1,4]thiazin-3-one par la 4*H*-[1,4]-benzoxazin-3-one.

### Préparation 8 : 3-{4-[2-(6-benzoyl-3-oxo-2,3-dihydro-4H-benzo[1,4]thiazin-4-yl)-éthoxy]-phényl}-2-éthoxy-propanoate d'éthyle

On procède comme dans la Préparation 5 en remplaçant le produit obtenu au stade A de la Préparation 2 par le produit obtenu au stade A de la Préparation 6.

### Préparation 9 : 3-{4-[2-(6-Benzoyl-3-oxo-2,3-dihydro-4H-benzo[1,4]thiazin-4-yl)-éthoxy]-phényl}-2-(2,2,2-trifluoroéthoxy)-propanoate d'éthyle

On procède comme dans la Préparation 4 en remplaçant le produit obtenu au stade A de la Préparation 1 par le produit obtenu au stade A de la Préparation 6.

### Préparation 10 : 3-{4-[2-(7-Benzoyl-3-oxo-2,3-dihydro-4H-benzo[1,4]oxazin-4-yl)-éthoxy]-phényl}-2-éthoxy-propanoate d'éthyle

On procède comme dans la Préparation 3 en remplaçant le produit obtenu au stade A de la Préparation 1 par le produit obtenu au stade A de la Préparation 7.

### Préparation 11 : 3-{4-[2-(7-Benzoyl-3-oxo-2,3-dihydro-4H-benzo[1,4]oxazin-4-yl)-éthoxy]-phényl}-2-(2,2,2-trifluoroéthoxy)-propanoate d'éthyle

On procède comme dans la Préparation 4 en remplaçant le produit obtenu au stade A de la Préparation 1 par le produit obtenu au stade A de la Préparation 7.

### Exemple 1 : 3-{4-[2-(7-(Méthoxyimino-phényl)-méthyl-3-oxo-2,3-dihydro-4H-benzo[1,4]thiazin-4-yl)-éthoxy]-phényl}-2-éthoxy-propanoate d'éthyle

Dans 30 ml de diméthylformamide, on dissoud 1g du composé obtenu dans la Préparation 1 puis on ajoute 0,92 g carbonate de potassium, et on laisse sous agitation à 80°C pendant une heure. Le 3-[4-(2-chloroéthoxy)-phényl]-2-éthoxy-propanoate d'éthyle (1,51 g) est dissout dans un minimum de diméthylformamide et on laisse sous agitation à 110°C pendant une nuit.
Le diméthylformamide est ensuite évaporé, puis la solution est hydrolysée et acidifiée. La phase aqueuse est extraite avec du dichlorométhane, séchée sur sulfate de magnésium, filtrée et la phase organique évaporée. Le résidu est purifié sur gel de silice avec du toluène et de l'acétate d'éthyle (8/2) comme éluant pour conduire au produit du titre sous la forme d'une huile.

| *Microanalyse élémentaire :* | | | | |
|---|---|---|---|---|
| | % | *C* | *H* | *N* |
| *Calculé :* | | *66,17* | *6,09* | *4,98* |
| *Trouvé :* | | *65,58* | *6,28* | *4,71* |

### Exemple 2 : 3-{4-[2-(7-(Méthoxyimino-phényl)-méthyl-3-oxo-2,3-dihydro-4H-benzo[1,4] thiazin-4-yl)-éthoxy]-phényl}-2-(2,2,2-trifluoroéthoxy)propanoate d'éthyle

Dans 30 ml de diméthylformamide, on dissoud 1 g du composé obtenu dans la Préparation 1 puis on ajoute 0,91 g carbonate de potassium, et on laisse sous agitation à 80°C pendant une heure. Le 3-[4-(2-chloroéthoxy)-phényl]-2-(2,2,2-trifluoroéthoxy)propanoate d'éthyle (1,78 g) est dissout dans un minimum de diméthylformamide et on laisse sous agitation à 110°C pendant une nuit.
Le diméthylformamide est ensuite évaporé, puis la solution est hydrolysée et acidifiée. La phase aqueuse est extraite avec du dichlorométhane, séchée sur sulfate de magnésium, filtrée et la phase organique évaporée. Le résidu est purifié sur gel de silice avec du toluène et de l'acétate d'éthyle (8/2) comme éluant pour conduire au produit du titre sous la forme d'une huile verte.

| *Microanalyse élémentaire :* | | | | |
|---|---|---|---|---|
| | % | *C* | *H* | *N* |
| *Calculé :* | | *60,38* | *5,07* | *4,54* |
| *Trouvé :* | | *60,79* | *5, 25* | *4,34* |

### Exemple 3 : 3-{4-[2-(6-(Méthoxyimino-phényl)-méthyl-3-oxo-2,3-dihydro-4H-benzo[1,4]oxazin-4-yl)-éthoxy]-phényl}-2-éthoxy-propanoate d'éthyle

Dans 30 ml de diméthylformamide, on dissoud 1 g du composé obtenu dans la Préparation 2 puis on ajoute 1 g de carbonate de potassium, et on laisse sous agitation à 80°C pendant une heure. Le 3-[4-(2-chloroéthoxy)-phényl]-2-éthoxy-propanoate d'éthyle (1,59 g) est dissout dans un minimum de diméthylformamide et on laisse sous agitation à 110°C pendant une nuit.
Le diméthylformamide est ensuite évaporé, puis la solution est hydrolysée et acidifiée. La phase aqueuse est extraite avec du dichlorométhane, séchée sur sulfate de magnésium, filtrée et la phase organique évaporée. Le résidu est purifié sur gel de silice avec du toluène et de l'acétate d'éthyle (8/2) comme éluant pour conduire au produit du titre.

### Exemple 4 : 3-{4-[2-(7-(Hydroxyimino-phényl)-méthyl-3-oxo-2,3-dihydro-4H-benzo[1,4]thiazin-4-yl)-éthoxy]-phényl}-2-éthoxy-propanoate d'éthyle

Dans 20 ml d'éthanol, on dissoud 1 g du composé obtenu dans la Préparation 3 puis on ajoute 0,4 g de chlorhydrate d'hydroxylamine et 0,6 g de pyridine. Le milieu est porté à reflux sous agitation pendant 4 heures.
On hydrolyse le milieu puis acidifie par de l'HCl 3N et la solution est extraite avec de l'acétate d'éthyle, séchée, filtrée et évaporée. Le résidu est purifié sur gel de silice avec du toluène et de l'acétate d'éthyle (8/2) comme éluant pour conduire au produit du titre sous la forme d'une huile.

| *Microanalyse élémentaire :* | | | | |
|---|---|---|---|---|
| | % | *C* | *H* | *N* |
| *Calculé :* | | *65, 67* | *5,88* | *5,11* |
| *Trouvé :* | | *65,85* | *6,27* | *5,63* |

### Exemple 5 : 3-{4-[2-(7-(Hydroxyimino-phényl)-méthyl-3-oxo-2,3-dihydro-4H-benzo[1,4]thiazin-4-yl)-éthoxy]-phényl}-2-(2,2,2-trifluoroéthoxy)-propanoate d'éthyle

Dans 20 ml d'éthanol, on dissoud 1 g du composé obtenu dans la Préparation 4 puis on ajoute 0,36 g de chlorhydrate d'hydroxylamine et 0,5 g de pyridine. Le milieu est porté à reflux sous agitation pendant 4 heures.
On hydrolyse le milieu puis acidifie par de l'HCl 3N et la solution est extraite avec de l'acétate d'éthyle, séchée, filtrée et évaporée. Le résidu est purifié sur gel de silice avec du toluène et de l'acétate d'éthyle (8/2) comme éluant pour conduire au produit du titre sous la forme d'une poudre verdâtre.

| *Microanalyse élémentaire :* | | | | |
|---|---|---|---|---|
| | % | *C* | *H* | *N* |
| *Calculé :* | | *59,79* | *4,85* | *4,65* |
| *Trouvé :* | | *60, 08* | *5,13* | *4, 28* |

### Exemple 6 : 3-{4-[2-(6-(Hydroxyimino-phényl)-méthyl-3-oxo-2,3-dihydro-4H-benzo[1,4]oxazin-4-yl)-éthoxy]-phényl}-2-éthoxy-propanoate d'éthyle

Dans 20 ml d'éthanol, on dissoud 1g du composé obtenu dans la Préparation 5 puis on ajoute 0,41 g de chlorhydrate d'hydroxylamine et 0,6 g de pyridine. Le milieu est porté à reflux sous agitation pendant 4 heures.
On hydrolyse le milieu puis acidifie par de l'HCl 3N et la solution est extraite avec de l'acétate d'éthyle, séchée, filtrée et évaporée. Le résidu est purifié sur gel de silice avec du toluène et de l'acétate d'éthyle (8/2) comme éluant pour conduire au produit du titre sous la forme d'une huile.

### Exemple 7 : Acide 3-{4-[2-(7-(hydroxyimino-phényl)-méthyl-3-oxo-2,3-dihydro-4H-benzo[1,4]thiazin-4-yl)-éthoxy]-phényl}-2-éthoxy-propanoïque

Dans un mélange tétrahydrofuranne/eau (12/8 ml), on dissoud 1 g du composé obtenu dans l'Exemple 4, puis on ajoute 0,13 g de lithine dissoute dans un minimum d'eau. La réaction est agitée à 50°C pendant une nuit.
On évapore ensuite le tétrahydrofuranne, puis la solution est hydrolysée, puis acidifiée par de l'HCl 3N. On extrait ensuite avec de l'acétate d'éthyle, sèche, filtre et évapore la phase organique. Le résidu est purifié sur gel de silice avec du dichlorométhane et du méthanol (95/5) comme éluant pour conduire au produit du titre sous la forme d'une poudre beige.
*Point de fusion : 74-76°C*

| *Microanalyse élémentaire :* | | | | |
|---|---|---|---|---|
| | *%* | *C* | *H* | *N* |
| *Calculé :* | | *64, 60* | *5,42* | *5,38* |
| *Trouvé :* | | *64,32* | *5,89* | *5, 02* |

### Exemple 8 : Acide 3-{4-[2-(7-(méthoxyimino-phényl)-méthyl-3-oxo-2,3-dihydro-4H-benzo[1,4]thiazin-4-yl)-éthoxy]-phényl}-2-éthoxy-propanoïque

Dans un mélange tétrahydrofuranne/eau (12/8 ml), on dissoud 1 g du composé obtenu dans l'Exemple 1, puis on ajoute 0,13 g de lithine dissoute dans un minimum d'eau. La réaction est agitée à 50°C pendant une nuit.
On évapore ensuite le tétrahydrofuranne, puis la solution est hydrolysée, puis acidifiée par de l'HCl 3N. On extrait ensuite avec de l'acétate d'éthyle, sèche, filtre et évapore la phase organique. Le résidu est purifié sur gel de silice avec du dichlorométhane et du méthanol (95/5) comme éluant pour conduire au produit du titre sous la forme d'une poudre jaunâtre.
*Point de fusion : 77-79°C*

| *Microanalyse élémentaire :* | | | | |
|---|---|---|---|---|
| | *%* | C | *H* | *N* |
| *Calculé :* | | *65,15* | *5,66* | *5,23* |
| *Trouvé :* | | *65,33* | *5,90* | *5,57* |

### Exemple 9 : Acide 3-{4-[2-(7-(hydroxyimino-phényl)-méthyl-3-oxo-2,3-dihydro-4H-benzo[1,4]thiazin-4-yl)-éthoxy]-phényl}-2-(2,2,2-trifluoroéthoxy)-propanoïque

Dans un mélange tétrahydrofuranne/eau (12/8 ml), on dissoud 1 g du composé obtenu dans l'Exemple 5, puis on ajoute 0,12 g de lithine dissoute dans un minimum d'eau. La réaction est agitée à 50°C pendant une nuit.
On évapore ensuite le tétrahydrofuranne, puis la solution est hydrolysée, puis acidifiée par de l'HCl 3N. On extrait ensuite avec de l'acétate d'éthyle, sèche, filtre et évapore la phase organique. Le résidu est purifié sur gel de silice avec du dichlorométhane et du méthanol (95/5) comme éluant pour conduire au produit du titre sous la forme d'une poudre verte.
*Point de fusion : 85-88°C*

| *Microanalyse élémentaire :* | | | | |
|---|---|---|---|---|
| | *%* | C | *H* | *N* |
| *Calculé :* | | *58,53* | *4,39* | *4,88* |
| *Trouvé :* | | *58,39* | *4,68* | *4,42* |

### Exemple 10 : Acide 3-{4-[2-(7-(méthoxyimino-phényl)-méthyl-3-oxo-2,3-dihydro-4H-benzo[1,4]thiazin-4-yl)-éthoxy]-phényl}-2-(2,2,2-trifluoroéthoxy)-propanoïque

Dans un mélange tétrahydrofuranne/eau (12/8 ml), on dissoud 1 g du composé obtenu dans l'Exemple 2, puis on ajoute 0,11 g de lithine dissoute dans un minimum d'eau. La réaction est agitée à 50°C pendant une nuit.
On évapore ensuite le tétrahydrofuranne, puis la solution est hydrolysée, puis acidifiée par de l'HCl 3N. On extrait ensuite avec de l'acétate d'éthyle, sèche, filtre et évapore la phase organique. Le résidu est purifié sur gel de silice avec du dichlorométhane et du méthanol (95/5) comme éluant pour conduire au produit du titre sous la forme d'une poudre verte.
*Point de fusion : 64-66°C*

| *Microanalyse élémentaire :* | | | | |
|---|---|---|---|---|
| | *%* | C | *H* | *N* |
| *Calculé :* | | *59,18* | *4, 62* | *4,76* |
| *Trouvé :* | | *59,53* | *4,31* | *4,54* |

### Exemple 11 : Acide 3-{4-[2-(6-(hydroxyimino-phényl)-méthyl-3-oxo-2,3-dihydro-4H-benzo[1,4]thiazin-4-yl)-éthoxy]-phényl}-2-éthoxy-propanoïque

Dans un mélange tétrahydrofuranne/eau (12/8 ml), on dissoud 1 g du composé obtenu dans l'Exemple 6, puis on ajoute 0,13 g de lithine dissoute dans un minimum d'eau. La réaction est agitée à 50°C pendant une nuit.
On évapore ensuite le tétrahydrofuranne, puis la solution est hydrolysée, puis acidifiée par de l'HCl 3N. On extrait ensuite avec de l'acétate d'éthyle, sèche, filtre et évapore la phase organique. Le résidu est purifié sur gel de silice avec du dichlorométhane et du méthanol (95/5) comme éluant pour conduire au produit du titre sous la forme d'une poudre beige.
*Point de fusion : 75-76°C*

| *Microanalyse élémentaire :* | | | | |
|---|---|---|---|---|
| | *%* | C | *H* | *N* |
| *Calculé :* | | *66,66* | *5,59* | *5,55* |
| *Trouvé :* | | *66,53* | *5,31* | *5,34* |

### Exemple 12 : Acide 3-{4-[2-(6-(méthoxyimino-phényl)-méthyl-3-oxo-2,3-dihydro-4H-benzo[1,4]thiazin-4-yl)-éthoxy]-phényl}-2-éthoxy-propanoïque

Dans un mélange tétrahydrofuranne/eau (12/8 ml), on dissoud 1 g du composé obtenu dans l'Exemple 3, puis on ajoute 0,13 g de lithine dissoute dans un minimum d'eau. La réaction est agitée à 50°C pendant une nuit.
On évapore ensuite le tétrahydrofuranne, puis la solution est hydrolysée, puis acidifiée par de l'HCl 3N. On extrait ensuite avec de l'acétate d'éthyle, séche, filtre et évapore la phase organique. Le résidu est purifié sur gel de silice avec du dichlorométhane et du méthanol (95/5) comme éluant pour conduire au produit du titre sous la forme d'une poudre jaunâtre.
*Point de fusion : 63-65°C*

| *Microanalyse élémentaire :* | | | | |
|---|---|---|---|---|
| | *%* | *C* | *H* | *N* |
| *Calculé :* | | *67,17* | *5,83* | *5,40* |
| *Trouvé :* | | *67,53* | *6,11* | *5,34* |

### Exemple 13 : 3-{4-[2-(6-(Méthoxyiminophényl)méthyl-3-oxo-2,3-dihydro-4H-benzo[1,4]thiazin-4-yl)-éthoxy]phényl}-2-éthoxy-propanoate d'éthyle

On procède comme dans l'Exemple 1 en remplaçant le composé de la Préparation 1 par le composé de la Préparation 6. Le produit du titre est obtenu sous la forme d'une huile.

| *Microanalyse élémentaire :* | | | | |
|---|---|---|---|---|
| | *%* | *C* | *H* | *N* |
| *Calculé :* | | *66,17* | *6,09* | *4,98* |
| *Trouvé :* | | *66,53* | *6,31* | *4,94* |

### Exemple 14 : 3-{4-[2-(6-(Méthoxyiminophény/)méthy/-3-oxo-2,3-dihydro-4H-benzo[1,4] thiazin-4-yl)éthoxy]phényl}-2-(2,2,2-trifluoroéthoxy)propanoate d'éthyle

On procède comme dans l'Exemple 2 en remplaçant le composé de la Préparation 1 par le composé de la Préparation 6. Le produit du titre est obtenu sous la forme d'une huile jaune.

| *Microanalyse élémentaire :* | | | | |
|---|---|---|---|---|
| | *%* | *C* | *H* | *N* |
| *Calculé :* | | *60,38* | *5,07* | *4,54* |
| *Trouvé :* | | *60,69* | *5,25* | *4,34* |

### Exemple 15 : 3-{4-[2-(7-(Méthoxyiminophényl)méthyl-3-oxo-2,3-dihydro-4H-benzo[1,4]thiazin-4-yl)-éthoxy]phényl}-2-éthoxy-propanoate d'éthyle

On procède comme dans l'Exemple 1 en remplaçant le composé de la Préparation 1 par le composé de la Préparation 7. Le produit du titre est obtenu sous la forme d'une huile.

| *Microanalyse élémentaire :* | | | | |
|---|---|---|---|---|
| | % | *C* | *H* | *N* |
| *Calculé :* | | *68,12* | *6,27* | *5,12* |

### Exemple 16 : 3-{4-[2-(7-(Méthoxyiminophényl)méthyl-3-oxo-2,3-dihydro-4H-benzo[1,4]thiazin-4-yl)-éthoxy]phényl}-2-(2,2,2-trifluroéthoxy) propanoate d'éthyle

On procède comme dans l'Exemple 2 en remplaçant le composé de la Préparation 1 par le composé de la Préparation 7. Le produit du titre est obtenu sous la forme d'une huile incolore.

| *Microanalyse élémentaire :* | | | | |
|---|---|---|---|---|
| | *%* | C | *H* | *N* |
| *Calculé :* | | *62,00* | *5,20* | *4,66* |
| *Trouvé :* | | *61,79* | *5,25* | *4,59* |

### Exemple 17 : 3-{4-[2-(6-(Hydroxyiminophényl)méthyl-3-oxo-2,3-dihydro-4H-benzo[1,4]thiazin-4-yl)-éthoxy]-phényl}-2-éthoxy-propanoate d'éthyle

On procède comme dans l'Exemple 4 en remplaçant le composé de la Préparation 3 par le composé de la Préparation 8. Le produit du titre est obtenu sous la forme d'une poudre jaune.
*Point de fusion : 65-66°C*

| *Microanalyse élémentaire :* | | | | |
|---|---|---|---|---|
| | *%* | *C* | *H* | *N* |
| *Calculé :* | | *65,67* | *5,88* | *5,11* |
| *Trouvé :* | | *65,89* | *5,60* | *5,75* |

### Exemple 18 :3-{4-[2-(6-(Hydroxyiminophényl)méthyl-3-oxo-2,3-dihydro-4H-benzo[1,4]thiazin-4-yl)-éthoxy]phényl}-2-(2,2,2-trifluoroéthoxy) propanoate d'éthyle

On procède comme dans l'Exemple 5 en remplaçant le composé de la Préparation 4 par le composé de la Préparation 9. Le produit du titre est obtenu sous la forme d'une poudre blanche.
*Point de fusion : 171-173°C*

| *Microanalyse élémentaire :* | | | | |
|---|---|---|---|---|
| | *%* | *C* | *H* | *N* |
| *Calculé :* | | *59,79* | *4,85* | *4, 65* |
| *Trouvé :* | | *60,01* | *5, 07* | *4,38* |

### Exemple 19 : 3-{4-[2-(7-(Hydroxyiminophényl)méthyl-3-oxo-2,3-dihydro-4H-benzo[1,4]thiazin-4-yl)-éthoxy]phényl}-2-éthoxy-propanoate d'éthyle

On procède comme dans l'Exemple 4 en remplaçant le composé de la Préparation 3 par le composé de la Préparation 10. Le produit du titre est obtenu sous la forme d'une poudre jaune.
*Point de fusion : 84-86°C*

| *Microanalyse élémentaire :* | | | | |
|---|---|---|---|---|
| | *%* | *C* | *H* | *N* |
| *Calculé :* | | *67,66* | *6,06* | *5,26* |
| *Trouvé :* | | *67,33* | *6,41* | *4,90* |

### Exemple 20 : 3-{4-[2-(7-(Hydroxyiminophényl)méthyl-3-oxo-2,3-dihydro-4H-benzo[1,4]thiazin-4-yl)-éthoxy]phényl}-2-(2,2,2-trifluoroéthoxy) propanoate d'éthyle

On procède comme dans l'Exemple 5 en remplaçant le composé de la Préparation 4 par le composé de la Préparation 11. Le produit du titre est obtenu sous la forme d'une poudre blanche.
*Point de fusion : 84-86°C*

| *Microanalyse élémentaire :* | | | | |
|---|---|---|---|---|
| | *%* | *C* | *H* | *N* |
| *Calculé :* | | *61,43* | *4,98* | *4,78* |
| *Trouvé :* | | *61, 08* | *5, 29* | *4,51* |

### Exemple 21 : Acide 3-{4-[2-(6-(méthoxyiminophényl)méthyl-3-oxo-2,3-dihydro-4H-benzo[1,4]thiazin-4-yl)-éthoxy]phényl}-2-éthoxypropanoïque

On procède comme dans l'Exemple 8 en remplaçant le composé de l'Exemple 1 par le composé de l'Exemple 13. Le produit du titre est obtenu sous la forme d'une poudre jaune.
*Point de fusion : 58-61 °C*

| *Microanalyse élémentaire :* | | | | |
|---|---|---|---|---|
| | *%* | *C* | *H* | *N* |
| *Calculé :* | | *65,03* | *5,83* | *5,23* |
| *Trouvé :* | | *65,13* | *5,61* | *5,34* |

### Exemple 22 :Acide 3-{4--[2-(6-(méthoxyiminophényl)méthyl-3-oxo-2,3-dihydro-4H-benzo[1,4]thiazin-4-yl)-éthoxy]phényl}-2-(2,2,2-trifluoroéthoxy) propanoïque

On procède comme dans l'Exemple 10 en remplaçant le composé de l'Exemple 2 par le composé de l'Exemple 14. Le produit du titre est obtenu sous la forme d'une poudre blanche.
*Point de fusion : 66-68°C*

| *Microanalyse élémentaire :* | | | | |
|---|---|---|---|---|
| | *%* | *C* | *H* | *N* |
| *Calculé :* | | *59,18* | *4, 62* | *4,76* |
| *Trouvé :* | | *58,79* | *4,71* | *4, 62* |

### Exemple 23 : Acide 3-{4-[2-(6-(hydroxyiminophényl)méthyl-3-oxo-2,3-dihydro-4H-benzo[1,4]thiazin-4-yl)-éthoxy]phényl}-2-éthoxypropanoïque

On procède comme dans l'Exemple 7 en remplaçant le composé de l'Exemple 4 par le composé de l'Exemple 17. Le produit du titre est obtenu sous la forme d'une poudre beige.
*Point de fusion : 127-130°C*

### Exemple 24 : Acide 3-{4-[2-(6-(hydroxyiminophényl)méthyl-3-oxo-2,3-dihydro-4H-benzo[1,4]thiazin-4-yl)-éthoxy]phényl}-2-(2,2,2-trifluoroéthoxy) propanoïque

On procède comme dans l'Exemple 9 en remplaçant le composé de l'Exemple 5 par le composé de l'Exemple 18. Le produit du titre est obtenu sous la forme d'une poudre blanche.
*Point de fusion : 97-99°C*

| *Microanalyse élémentaire :* | | | | |
|---|---|---|---|---|
| | *%* | *C* | *H* | *N* |
| *Calculé :* | | *58,53* | *4,39* | *4,88* |
| *Trouvé :* | | *58,39* | *4, 68* | *4,72* |

### Exemple 25 : Acide 3-{4-[2-(7-(méthoxyiminophényl)méthyl-3-oxo-2,3-dihydro-4H-benzo[1,4]thiazin-4-yl)-éthoxy]phényl}-2-éthoxypropanoïque

On procède comme dans l'Exemple 8 en remplaçant le composé de l'Exemple 1 par le composé de l'Exemple 15. Le produit du titre est obtenu sous la forme d'une poudre blanche.
*Point de fusion : 54-57°C*

| *Microanalyse élémentaire :* | | | | |
|---|---|---|---|---|
| | *%* | *C* | *H* | *N* |
| *Calculé :* | | *67,17* | *5,83* | *5,40* |
| *Trouvé :* | | *67,20* | *6,08* | *5,21* |

### Exemple 26 : Acide 3-{4-[2-(7-(méthoxyiminophényl)méthyl-3-oxo-2,3-dihydro-4H-benzo[1,4]thiazin-4-yl)-éthoxy]phényl}-2-(2,2,2-trifluoroéthoxy) propanoïque

On procède comme dans l'Exemple 10 en remplaçant le composé de l'Exemple 2 par le composé de l'Exemple 16. Le produit du titre est obtenu sous la forme d'une poudre jaune.
*Point de fusion : 61-64°C*

| *Microanalyse élémentaire :* | | | | |
|---|---|---|---|---|
| | *%* | *C* | *H* | *N* |
| *Calculé :* | | *60,84* | *4,75* | *4,89* |
| *Trouvé :* | | *60,53* | *4,41* | *4,64* |

### Exemple 27 : Acide 3-{4-[2-(7-(hydroxyiminophényl)méthyl-3-oxo-2,3-dihydro-4H-benzo[1,4]thiazin-4-yl)-éthoxy]phényl}-2-éthoxypropanoïque

On procède comme dans l'Exemple 7 en remplaçant le composé de l'Exemple 4 par le composé de l'Exemple 19. Le produit du titre est obtenu sous la forme d'une poudre blanche.
*Point de fusion : 95-97°C*

| *Microanalyse élémentaire :* | | | | |
|---|---|---|---|---|
| | % | C | *H* | *N* |
| *Calculé :* | | *66,66* | *5,59* | *5,55* |
| *Trouvé :* | | *66,53* | *5,31* | *5,34* |

### Exemple 28 : Acide 3-{4-[2-(7-(hydroxyiminophényl)méthyl-3-oxo-2,3-dihydro-4H-benzo[1,4]thiazin-4-yl)-éthoxy]phényl}-2-(2,2,2-trifluoroéthoxy) propanoïque

On procède comme dans l'Exemple 9 en remplaçant le composé de l'Exemple 5 par le composé de l'Exemple 20. Le produit du titre est obtenu sous la forme d'une poudre blanche.
*Point de fusion : 86-88°C* .

| *Microanalyse élémentaire :* | | | | |
|---|---|---|---|---|
| | *%* | *C* | *H* | *N* |
| *Calculé :* | | *60,22* | *4,51* | *5,02* |
| *Trouvé :* | | *59,86* | *4,74* | *4,81* |

### ETUDE PHARMACOLOGIQUE

### Exemple A : Etude de la toxicité aiguë

La toxicité aiguë a été appréciée après administration orale à des lots de 8 souris (26 ± 2 g). Les animaux ont été observés à intervalles réguliers au cours de la première journée et quotidiennement pendant deux semaines suivant le traitement. La DL₅₀, entraînant la mort de 50 % des animaux, a été évaluée et a montré la faible toxicité des composés de l'invention.

### Exemple B : Efficacité dans les modèles génétiques

Des mutations chez des animaux de laboratoire ainsi que des sensibilités différentes à des régimes alimentaires ont permis le développement de modèles animaux présentant des diabètes non insulino-dépendants et des hyperlipidémies associés à l'obésité et à la résistance à l'insuline.
Des modèles génétiques souris (ob/ob) (Diabetes, 1982, 31 (1), 1-6) et rats Zucker (fa/fa) ont été développés par différents laboratoires pour comprendre la physiopathologie de ces maladies et tester l'efficacité de nouveaux composés antidiabétiques (Diabetes, 1983, 32, 830-838).

### Effet antidiabétique et hypolipémiant chez la souris ob/ob

La souris femelle ob/ob (Harlan) âgée de 10 semaines est utilisée pour les tests *in vivo*. Ces animaux sont maintenus sous un cycle lumière-obscurité de 12 heures à 25 °C. Cette souris a une hyperglycémie basale située à 2 g/l. Les animaux sont randomisés par rapport à leur glycémie pour former des groupes de six. Les composés testés par voie intrapéritonéale sont dissous dans un mélange de diméthylsulfoxide (10 %) et de solutol (15 %) pour être administrés à 10 mg/kg sous un volume de 2,5 ml/kg, deux fois par jour pendant quatre jours. Par voie *per os*, les composés sont testés à 30 mg/kg administrés sous un volume de 2,5 ml/kg de HEC 1 %, deux fois par jour pendant quatre jours. Les groupes contrôles reçoivent les solvants dans les mêmes conditions que les groupes traités. L'activité des produits est évaluée par une mesure de la glycémie, des triglycérides et de l'insulinémie 24 heures après la dernière administration et par la mesure quotidienne du poids corporel.

Les composés de l'invention montrent une très bonne capacité à réduire la glycémie, les triglycérides et l'insulinémie : à titre d'exemple, le composé de l'Exemple 11 administré à la dose de 3 mg/kg montre une réduction de 25% de la glycémie, de 45% des triglycérides et de 22% de l'insulinémie, avec une variation du poids corporel non significative, alors que dans les mêmes conditions, la Rosiglitazone montre une augmentation significative en quatre jours. Par ailleurs, aucun effet secondaire n'a été observé durant les test *in vivo*.

### Exemple C : Composition pharmaceutique

| | |
|---|---|
| 1000 comprimés dosés à 5 mg de l'acide 3-{4-[2-(6-(hydroxyimino-phényl)-méthyl-3-oxo-2,3-dihydro-4*H*-benzo[1,4]oxazin-4-yl)-éthoxy]-phényl}-2-éthoxy-propanoïque (Exemple 11) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

### Exemple D : Variation du poids corporel

Des souris mâles C57 Black 6 ob/ob de 8 à 12 semaines ont été utilisées. Après mise en quarantaine d'une semaine, elles ont été pesées puis randomisées en fonction de leur poids, et 6 groupes homogènes (poids de départ non significativement différent) ont été formés. Après avoir été pesées, les différentes associations à tester sont injectées par voie intrapéritonéale une fois par jour pendant 7 jours. Les molécules sont injectées dans une solution DMSO 5 % / Solutol 15 % / Qsp H₂O chauffée à 65°C pour assurer une bonne dissolution. La solution est de plus préchauffée avant injection. Les souris sont pesées tous les jours et le poids obtenu après 7 jours de traitement est relevé.
Les résultats obtenus montrent clairement :
- que l'association selon l'invention entre un composé de formule (I) et un agent antioxydant permet de réduire significativement le poids des souris obèses,
- qu'il existe une synergie entre les 2 composants de l'association, la perte de poids constatée étant bien supérieure avec l'association qu'avec chaque composant administré seul.

### Exemple E : Composition pharmaceutique

100 comprimés dosés à 30 mg de l'acide 3-{4-[2-(6-(hydroxyimino-phényl)-méthyl-3-oxo-2,3-dihydro-4*H-*benzo[1,4]oxazin-4-yl)-éthoxy]-phényl}-2-éthoxy-propanoïque et 10 mg de coenzyme Q₁₀

| | |
|---|---|
| Acide 3-{4-[2-(6-(hydroxyimino-phényl)-méthyl-3-oxo-2,3-dihydro-4*H-*benzo[1,4]oxazin-4-yl)-éthoxy]-phényl}-2-éthoxy-propanoïque (Exemple 11) | 3 g |
| Coenzyme Q₁₀ | 1 g |
| Amidon de blé | 20g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 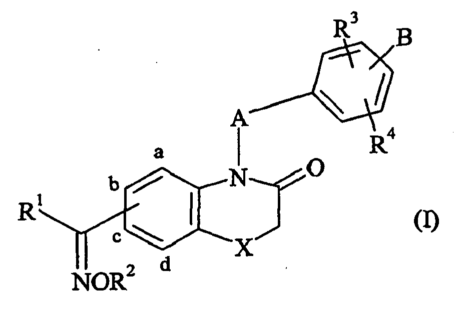 dans laquelle :
• X représente un atome d'oxygène ou de soufre,
• A représente une chaîne alkylène (C₁-C₆) dont un groupement CH₂ peut être optionnellement remplacé par un hétéroatome choisi parmi oxygène ou soufre, ou par un groupement NRₐ (où Rₐ représente un atome d'hydrogène ou un groupement alkyle (C₁-C₆) linéaire ou ramifié), ou par un groupement phénylène ou naphtylène,
• R¹ et R², identiques ou différents, représentent un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié,
• R³ et R⁴, identiques ou différents, représentent un atome d'hydrogène ou d'halogène, ou un groupement R, OR ou NRR', où R et R', identiques ou différents, représentent un atome d'hydrogène ou un groupement alkyle (C₁-C₆) linéaire ou ramifié, alkényle (C₂-C₆) linéaire ou ramifié, alkynyle (C₂-C₆) linéaire
ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié,
ou R³ et R⁴ forment ensemble avec les atomes de carbone qui les portent, lorsqu'ils sont portés par deux atomes de carbone adjacents, un cycle comportant 5 ou 6 chaînons et pouvant optionnellement contenir un hétéroatome choisi parmi oxygène, soufre et azote,
• B représente un groupement alkyle (C₁-C₆) linéaire ou ramifié, ou alkényle (C₂-C₆) linéaire ou ramifié, ces groupements étant substitués :
◆ par un groupement de formule (II) : 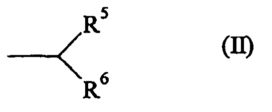
dans laquelle :
* R⁵ représente un groupement 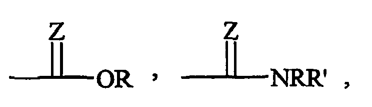 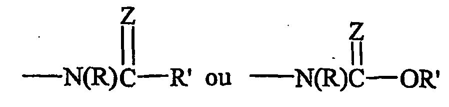
dans lesquels Z représente un atome d'oxygène ou de soufre et R et R', identiques ou différents, sont tels que définis précédemment,
* et R⁶ représente un groupement aryle, arylalkyle dont la partie alkyle contient de 1 à 6 atomes de carbone et peut être linéaire ou ramifiée, hétéroaryle, hétéroarylalkyle dont la partie alkyle contient 1 à 6 atomes de carbone et peut être linéaire ou ramifié, CN, Tétrazole, -OR, -NRR' , 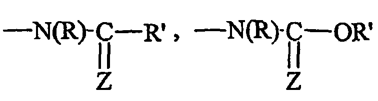
ou 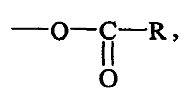 dans lesquels Z est tel que défini précédemment et R et R', identiques ou différents, peuvent prendre les mêmes valeurs que défini précédemment,
◆ ou par un groupement R⁷ où R⁷ représente un groupement CN, tétrazole, 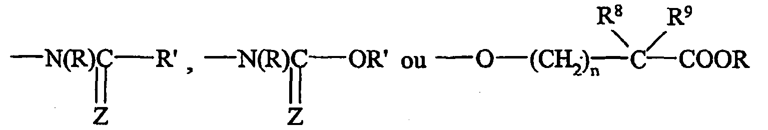
dans lesquels Z est tel que défini précédemment et R et R', identiques ou différents, peuvent prendre les mêmes valeurs que défini précédemment, n représente 0, 1, 2, 3, 4, 5 ou 6, et R⁸ et R⁹, identiques ou différents, représentent un atome d'hydrogène ou un groupement alkyle (C₁-C₆) linéaire ou ramifié étant entendu que R⁸ et R⁹ ne peuvent représenter simultanément un atome d'hydrogène,
ou B représente un groupement de formule (II) ou un groupement R⁷ tels que définis précédemment,
étant entendu que :
* l'oxime R¹-C(=N-OR²)- peut être de configuration Z ou E,
* par aryle on entend un groupement phényle, naphtyle ou biphényle, ces groupements pouvant être optionnellement partiellement hydrogénés,
* par hétéroaryle on entend tout groupement aromatique mono ou bicyclique contenant 5 à 10 chaînons, pouvant être optionnellement partiellement hydrogéné sur un des cycles dans le cas des hétéroaryles bicycliques, et contenant 1 à 3 hétéroatomes choisis parmi oxygène, azote et soufre,
les groupements aryle et hétéroaryle ainsi définis pouvant être optionnellement substitués par 1 à 3 groupements, identiques ou différents, choisis parmi alkyle (C₁-C₆) linéaire ou ramifié, polyhalogénoalkyle (C₁-C₆) linéaire ou ramifié, alkoxy (C₁-C₆) linéaire ou ramifié, hydroxy, carboxy, formyle, acyle (C₁-C₆) linéaire ou ramifié, aroyle, NR_{b}R_{c} (dans lequel R_{b} et R_{c}, identiques ou différents, représentent un atome d'hydrogène, un groupement alkyle (C₁-C₆) linéaire ou ramifié, aryle ou hétéroaryle), ester, amido, nitro, cyano, ou atomes d'halogène,
leurs énantiomères et diastéréoisomères ainsi que leurs sels d'addition à un acide ou à une base pharmaceutiquement acceptables.

2. Composés de formule (I) selon la revendication 1 pour lesquels le groupement R¹-C(=N-OR²)- est en position b ou c, leurs énantiomères et diastéréoisomères ainsi que leurs sels d'addition à un acide ou à une base pharmaceutiquement acceptables.

3. Composés de formule (I) selon la revendication 1 pour lesquels R³ et R⁴ représentent un atome d'hydrogène, leurs énantiomères et diastéréoisomères ainsi que leurs sels d'addition à un acide ou à une base pharmaceutiquement acceptables.

4. Composés de formule (I) selon la revendication 1 pour lesquels A représente un groupement éthylèneoxy, leurs énantiomères et diastéréoisomères ainsi que leurs sels d'addition à un acide ou à une base pharmaceutiquement acceptables.

5. Composés de formule (I) selon la revendication 1 pour lesquels R² représente un atome d'hydrogène, leurs énantiomères et diastéréoisomères ainsi que leurs sels d'addition à un acide ou à une base pharmaceutiquement acceptables.

6. Composés de formule (I) selon la revendication 1 pour lesquels R² représente un groupement alkyle, leurs énantiomères et diastéréoisomères ainsi que leurs sels d'addition à un acide ou à une base pharmaceutiquement acceptables.

7. Composés de formule (I) selon la revendication 1 pour lesquels R¹ représente un groupement phényle non substitué ou substitué, leurs énantiomères et diastéréoisomères ainsi que leurs sels d'addition à un acide ou à une base pharmaceutiquement acceptables.

8. Composés de formule (I) selon la revendication 1 pour lesquels B représente un groupement alkyle substitué par un groupement 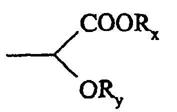 dans lequel Rₓ et R_{y}, identiques ou différents représentent un atome d'hydrogène ou un groupement alkyle, leurs énantiomères et diastéréoisomères ainsi que leurs sels d'addition à un acide ou à une base pharmaceutiquement acceptables.

9. Composés de formule (I) selon la revendication 1 pour lesquels B représente un groupement 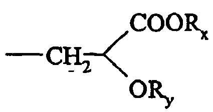 dans lequel Rₓ et R_{y}, identiques ou différents représentent un atome d'hydrogène ou un groupement alkyle, leurs énantiomères et diastéréoisomères ainsi que leurs sels d'addition à un acide ou à une base pharmaceutiquement acceptables.

10. Composés de formule (I) selon la revendication 1 qui sont le 2-éthoxy-3-[4-(2-{7-[(méthoxyimino)(phényl)méthyl]-3-oxo-2,3-dihydro-4*H*-1,4-benzothiazin-4-yl} éthoxy)phényl]propanoate d'éthyle, le 3-[4-(2-{7-[(méthoxyimino)(phényl)méthyl]-3-oxo-2,3-dihydro-4*H*-1,4-benzothiazin-4-yl}éthoxy)phényl]-2-(2,2,2-trifluoroéthoxy) propanoate d'éthyle, le 2-éthoxy-3-[4-(2-{6-[(méthoxyimino)(phényl)méthyl]-3-oxo-2,3-dihydro-4*H*-1,4-benzoxazin-4-yl}éthoxy)phényl]propanoate d'éthyle, le 2-éthoxy-3-[4-(2-{7-[(hydroxyimino)(phényl)méthyl]-3-oxo-2,3-dihydro-4*H*-1,4-benzothiazin-4-yl}éthoxy)phényl]propanoate d'éthyle, le 3-[4-(2-{7-[(hydroxyimino)(phényl) méthyl]-3-oxo-2,3-dihydro-4*H*-1,4-benzothiazin-4-yl}éthoxy)phényl]-2-(2,2,2-trifluoroéthoxy)propanoate d'éthyle, le 2-éthoxy-3-[4-(2-{6-[(hydroxyimino) (phényl)méthyl]-3-oxo-2,3-dihydro-4*H*-1,4-benzoxazin-4-yl}éthoxy)phényl] propanoate d'éthyle, l'acide 2-éthoxy-3-[4-(2-{7-[(hydroxyimino)(phényl)méthyl]-3-oxo-2,3-dihydro-4*H*-1,4-benzothiazin-4-yl}éthoxy)phényl]propanoïque, l'acide 2-éthoxy-3-[4-(2-{7-[(méthoxyimino)(phényl)méthyl]-3-oxo-2,3-dihydro-4*H*-1,4-benzo thiazin-4-yl}éthoxy)phényl]propanoïque, l'acide 3-[4-(2-{7-[(hydroxyimino)(phényl) méthyl]-3-oxo-2,3-dihydro-4*H*-1,4-benzothiazin-4-yl}éthoxy)phényl]-2-(2,2,2-trifluoroéthoxy)propanoïque, l'acide 3-[4-(2-{7-[(méthoxyimino)(phényl)méthyl]-3-oxo-2,3-dihydro-4*H*-1,4-benzothiazin-4-yl}éthoxy)phényl]-2-(2,2,2-trifluoroéthoxy) propanoïque, l'acide 2-éthoxy-3-[4-(2-{6-[(hydroxyimino)(phényl)méthyl]-3-oxo-2,3-dihydro-4*H*-1,4-benzoxain-4-yl}éthoxy)phényl]propanoïque, l'acide 2-éthoxy-3-[4-(2- {6-[(méthoxyimino)(phényl)méthyl]-3-oxo-2,3-dihydro-4*H*-1,4-benzoxazin-4-yl}éthoxy)phényl]propanoïque, leurs énantiomères et diastéréoisomères ainsi que leurs sels d'addition à un acide ou à une base pharmaceutiquement acceptables.

11. Procédé de préparation des composés de formule (I) selon la revendication 1 **caractérisé en ce que** l'on utilise comme produit de départ un composé de formule (III) : 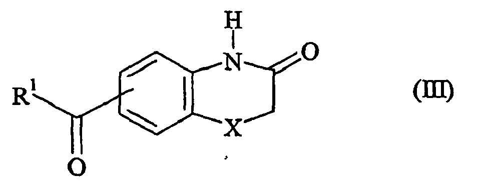 dans laquelle R¹ et X sont tels que définis dans la formule (I),
sur lequel on condense en milieu basique un composé de formule (IV) : 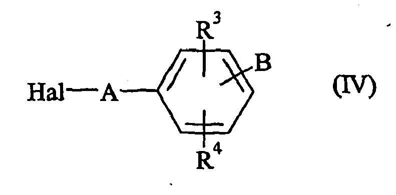 dans laquelle A, B, R³ et R⁴ sont tels que définis dans la formule (I) et Hal représente un atome d'halogène, pour conduire au composé de formule (V) : 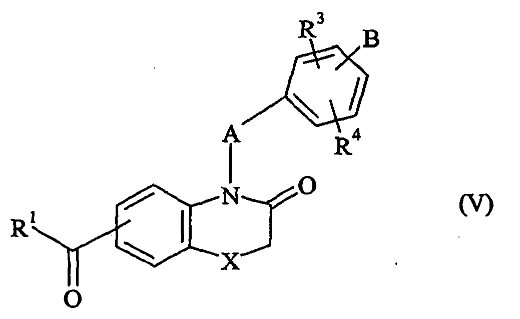 dans laquelle R¹, R³, R⁴, A, B et X sont tels que définis dans la formule (I),
que l'on soumet à l'action d'un composé de formule R²O-NH₂ dans laquelle R² est tel que défini dans la formule (I) pour conduire au composé de formule (I) : 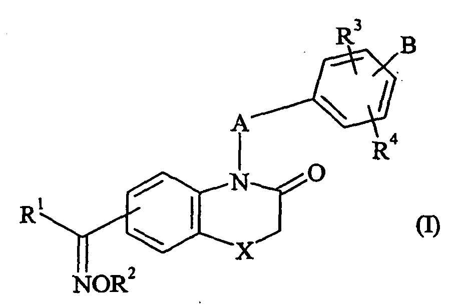 qui peut être purifié selon une technique classique de séparation; que l'on transforme, si on le souhaite en ses sels d'addition à un acide ou à une base pharmaceutiquement acceptable et dont on sépare éventuellement les isomères selon une technique classique de séparation.

12. Procédé de préparation des composés de formule (I) selon la revendication 1 **caractérisé en ce que** l'on utilise comme produit de départ un composé de formule (III) : 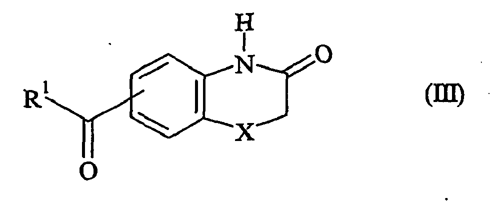 dans laquelle R¹ et X sont tels que définis dans la formule (I),
sur lequel on condense un composé de formule R²O-NH₂ dans laquelle R² est tel que défini dans la formule (I) pour conduire au composé de formule (VI) : 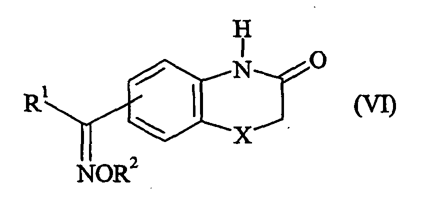 dans laquelle R¹, R² et X sont tels que définis dans la formule (I),
sur lequel on condense en milieu basique un composé de formule (IV) : 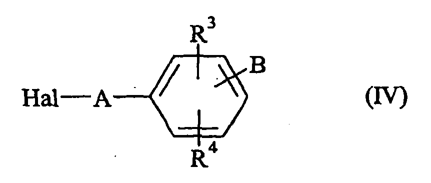 dans laquelle A, B, R³ et R⁴ sont tels que définis dans la formule (I) et Hal représente un atome d'halogène, pour conduire au composé de formule (I) : 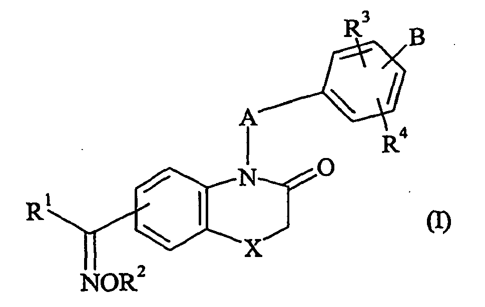 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

13. Compositions pharmaceutiques contenant comme principe actif au moins un composé de formule (I) selon l'une quelconque des revendications 1 à 10 ou un de ses sels d'addition à un acide ou à une base pharmaceutiquement acceptables, seul ou en combinaison avec un ou plusieurs excipients pharmaceutiquement acceptables.

14. Compositions pharmaceutiques selon la revendication 13 utiles pour la fabrication d'un médicament pour le traitement et/ou la prophylaxie des hyperglycémies, des dyslipidémies, et plus particulièrement dans le traitement des diabètes non insulino-dépendants de type II, de la résistance à l'insuline, de l'intolérance au glucose, des désordres reliés au syndrome X, des maladies artérielles coronaires et d'autres maladies cardiovasculaires, des maladies rénales, des rétinopathies, des désordres reliés à l'activation des cellules endothéliales, du psoriasis, du syndrome polycystique ovarien, de la démence, de l'ostéoporose, des maladies inflammatoires intestinales, des dystrophies myotoniques, des pancréatites, de l'artériosclérose, du xanthome, mais également dans le traitement ou la prévention du diabète de type I, de l'obésité, de la régulation de l'appétit, de l'anorexie, de la boulimie, de l'anorexie nerveuse, ainsi que des pathologies cancéreuses et notamment les cancers hormono-dépendants tels que le cancer du sein et le cancer du colon, et en tant qu'inhibiteurs d'angiogénèse.

15. Association contenant un composé de formule (I) selon l'une des revendications 1 à 10 et un agent antioxydant.

16. Association selon la revendication 15 dans laquelle le composé de formule (I) est l'acide 3-{4-[2-(6-(hydroxyimino-phényl)-méthyl-3-oxo-2,3-dihydro-4*H*-benzo[1,4] oxazin-4-yl)-éthoxy]-phényl}-2-éthoxy-propanoïque, ses énantiomères et diastéréoisomères ainsi que ses sels d'addition à un acide ou à une base pharmaceutiquement acceptables.

17. Association selon la revendication 15 ou 16 dans laquelle l'agent antioxydant est le coenzyme Q₁₀.

18. Association selon la revendication 15 ou 16 dans laquelle l'agent antioxydant est la vitamine E.

19. Association selon la revendication 15 ou 16 qui est l'acide 3-{4-[2-(6-(hydroxyimino-phényl)-méthyl-3-oxo-2,3-dihydro-4*H*-benzo[1,4]oxazin-4-yl)-éthoxy]-phényl}-2-éthoxy-propanoïque et le coenzyme Q₁₀.

20. Compositions pharmaceutiques contenant comme principe actif une association selon l'une quelconque des revendications 15 à 19 seule ou en combinaison avec un ou plusieurs excipients pharmaceutiquement acceptables.

21. Compositions pharmaceutiques selon la revendication 20 utiles pour la fabrication d'un médicament pour le traitement et/ou la prévention de l'obésité.

22. Compositions pharmaceutiques selon la revendication 20 utiles pour la fabrication d'un médicament pour le traitement et/ou la prévention des surcharges pondérales **caractérisées par** un index de poids corporel supérieur à 25 et inférieur à 30.

23. Utilisation d'une association selon l'une quelconque des revendications 15 à 19 pour l'obtention de compositions pharmaceutiques destinées au traitement et/ou à la prévention de l'obésité.

24. Utilisation d'une association selon l'une quelconque des revendications 15 à 19 pour l'obtention de compositions pharmaceutiques destinées au traitement et/ou à la prévention de l'obésité induite par un traitement thérapeutique.

25. Utilisation d'une association selon l'une quelconque des revendications 15 à 19 pour l'obtention de compositions pharmaceutiques destinées au traitement et/ou à la prévention de l'obésité induite par un traitement du diabète de type I ou II.

26. Utilisation d'une association selon l'une quelconque des revendications 15 à 19 pour l'obtention de compositions pharmaceutiques destinées au traitement et/ou à la prévention des surcharges pondérales **caractérisées par** un index de poids corporel supérieur à 25 et inférieur à 30.

27. Utilisation d'une association selon l'une quelconque des revendications 15 à 19 pour l'obtention de compositions pharmaceutiques destinées au traitement et/ou à la prévention des surcharges pondérales **caractérisées par** un index de poids corporel supérieur à 25 et inférieur à 30 induites par un traitement thérapeutique.

28. Utilisation d'une association selon l'une quelconque des revendications 15 à 19 pour l'obtention de compositions pharmaceutiques destinées au traitement et/ou à la prévention des surcharges pondérales **caractérisées par** un index de poids corporel supérieur à 25 et inférieur à 30 induites par un traitement du diabète de type I ou II.

## Claims

1. Compounds of formula (1) : 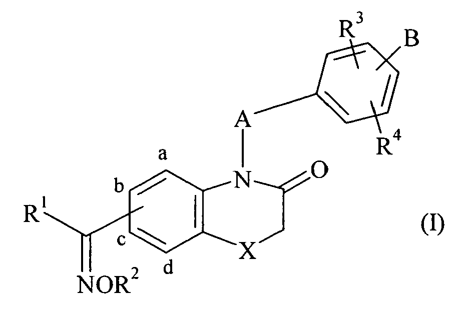 wherein :
• X represents an oxygen atom or a sulphur atom,
• A represents a (C₁-C₆)alkylene chain in which a CH₂ group may optionally be replaced by a hetero atom selected from oxygen and sulphur, or by an NRₐ group (wherein Rₐ represents a hydrogen atom or a linear or branched (C₁-C₆)alkyl group), or by a phenylene or naphthylene group,
• R¹ and R², which may be identical or different, each represents a hydrogen atom, a linear or branched (C₁-C₆)alkyl group, a linear or branched (C₂-C₆)alkenyl group, a linear or branched (C₂-C₆)alkynyl group, an aryl group, an aryl-(C₁-C₆)alkyl group in which the alkyl moiety may be linear or branched, an aryl-(C₂-C₆)alkenyl group in which the alkenyl moiety may be linear or branched, an aryl-(C₂-C₆)alkynyl group in which the alkynyl moiety may be linear or branched, a heteroaryl group, a heteroaryl-(C₁-C₆)alkyl group in which the alkyl moiety may be linear or branched, a heteroaryl-(C₂-C₆)alkenyl group in which the alkenyl moiety may be linear or branched, a heteroaryl-(C₂-C₆)alkynyl group in which the alkynyl moiety may be linear or branched, a (C₃-C₈)cycloalkyl group, a (C₃-C₈)cycloalkyl-(C₁-C₆)alkyl group in which the alkyl moiety may be linear or branched or a linear or branched (C₁-C₆)poly-haloalkyl group,
• R³ and R⁴, which may be identical or different, each represents a hydrogen atom, a halogen atom, or an R, OR or NRR' group wherein R and R', which may be identical or different, each represents a hydrogen atom or a linear or branched (C₁-C₆)alkyl group, a linear or branched (C₂-C₆)alkenyl group, a linear or branched (C₂-C₆)alkynyl group, an aryl group, an aryl-(C₁-C₆)alkyl group in which the alkyl moiety may be linear or branched, an aryl-(C₂-C₆)alkenyl group in which the alkenyl moiety may be linear or branched, an aryl-(C₂-C₆)alkynyl group in which the alkynyl moiety may be linear or branched, a heteroaryl group, a heteroaryl-(C₁-C₆)alkyl group in which the alkyl moiety may be linear or branched, a heteroaryl-(C₂-C₆)alkenyl group in which the alkenyl moiety may be linear or branched, a heteroaryl-(C₂-C₆)alkynyl group in which the alkynyl moiety may be linear or branched, a (C₃-C₈)cycloalkyl group, a (C₃-C₈)cycloalkyl-(C₁-C₆)alkyl group in which the alkyl moiety may be linear or branched or a linear or branched (C₁-C₆)polyhaloalkyl group,
or R³ and R⁴, together with the carbon atoms carrying them, when they are carried by two adjacent carbon atoms, form a ring that comprises 5 or 6 ring members and that may optionally contain a hetero atom selected from oxygen, sulphur and nitrogen,
• B represents a linear or branched (C₁-C₆)alkyl group or a linear or branched (C₂-C₆)alkenyl group, those groups being substituted :
◆ by a group of formula (II) : 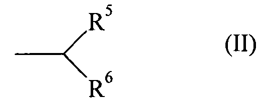
wherein :
* R⁵ represents a 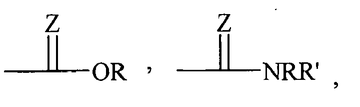 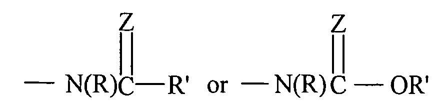 group wherein Z represents an oxygen atom or a sulphur atom and R and R', which may be identical or different, are as defined hereinbefore,
* and R⁶ represents an aryl group, an arylalkyl group in which the alkyl moiety contains from 1 to 6 carbon atoms and may be linear or branched, a heteroaryl group, a heteroarylalkyl group in which the alkyl moiety contains from 1 to 6 carbon atoms and may be linear or branched, CN, tetrazole -OR, -NRR', 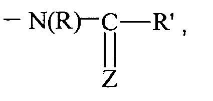 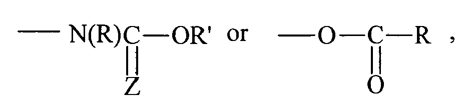
wherein Z is as defined hereinbefore and R and R', which may be identical or different, have the same definitions as given hereinbefore,
◆ or by a group R⁷, R⁷ representing a CN, tetrazole, 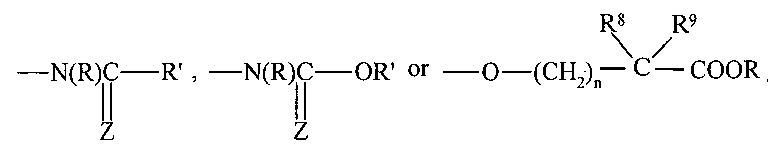 group
wherein Z is as defined hereinbefore and R and R', which may be identical or different, have the same definitions as given hereinbefore, n represents 0, 1, 2, 3, 4, 5 or 6, and R⁸ and R⁹, which may be identical or different, each represents a hydrogen atom or a linear or branched (C₁-C₆)alkyl group, wherein R⁸ and R⁹ cannot simultaneously represent a hydrogen atom,
or B represents a group of formula (II) or a group R⁷ as defined hereinbefore, it being understood that :
* the oxime R¹-C(=N-OR²)- may have the Z or E configuration,
* aryl is understood to mean a phenyl, naphthyl or biphenyl group, wherein those groups may optionally be partially hydrogenated,
* heteroaryl is understood to mean any aromatic mono- or bi-cyclic group containing from 5 to 10 ring members, which in the case of bicyclic heteroaryl groups may optionally be partially hydrogenated on one of the rings, and containing from 1 to 3 hetero atoms selected from oxygen, nitrogen and sulphur,
wherein the aryl and heteroaryl groups so defined may optionally be substituted by from 1 to 3 identical or different groups selected from linear or branched (C₁-C₆)alkyl, linear or branched (C₁-C₆)polyhaloalkyl, linear or branched (C₁-C₆)alkoxy, hydroxy, carboxy, formyl, linear or branched (C₁-C₆)acyl, aroyl, NR_{b}R_{c} (wherein R_{b} and R_{c}, which may be identical or different, each represents a hydrogen atom, a linear or branched (C₁-C₆)alkyl group, an aryl group or a heteroaryl group), ester, amido, nitro, cyano, and halogen atoms,
enantiomers and diastereoisomers thereof, and also pharmaceutically acceptable addition salts thereof with an acid or a base.

2. Compounds of formula (I) according to claim 1 wherein the group R¹-C(=N-OR²)- is in the b or c position, enantiomers and diastereoisomers thereof and also pharmaceutically acceptable addition salts thereof with an acid or a base.

3. Compounds of formula (I) according to claim 1 wherein R³ and R⁴ represent a hydrogen atom, enantiomers and diastereoisomers thereof and also pharmaceutically acceptable addition salts thereof with an acid or a base.

4. Compounds of formula (I) according to claim 1 wherein A represents an ethyleneoxy group, enantiomers and diastereoisomers thereof and also pharmaceutically acceptable addition salts thereof with an acid or a base.

5. Compounds of formula (I) according to claim 1 wherein R² represents a hydrogen atom, enantiomers and diastereoisomers thereof and also pharmaceutically acceptable addition salts thereof with an acid or a base.

6. Compounds of formula (I) according to claim 1 wherein R² represents an alkyl group, enantiomers and diastereoisomers thereof and also pharmaceutically acceptable addition salts thereof with an acid or a base.

7. Compounds of formula (I) according to claim 1 wherein R¹ represents an unsubstituted or substituted phenyl group, enantiomers and diastereoisomers thereof and also pharmaceutically acceptable addition salts thereof with an acid or a base.

8. Compounds of formula (I) according to claim 1 wherein B represents an alkyl group substituted by a group 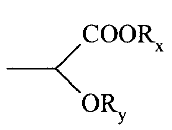 wherein Rₓ and R_{y}, which may be identical or different, each represents a hydrogen atom or an alkyl group, enantiomers and diastereoisomers thereof and also pharmaceutically acceptable addition salts thereof with an acid or a base.

9. Compounds of formula (I) according to claim 1 wherein B represents a group 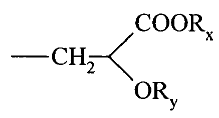 wherein Rₓ and R_{y}, which may be identical or different, each represents a hydrogen atom or an alkyl group, enantiomers and diastereoisomers thereof and also pharmaceutically acceptable addition salts thereof with an acid or a base.

10. Compounds of formula (I) according to claim 1 which are ethyl 2-ethoxy-3-[4-(2-{7-[(methoxyimino)(phenyl)methyl]-3-oxo-2,3-dihydro-4*H*-1,4-benzothiazin-4-yl}-ethoxy)phenyl]propanoate, ethyl 3-[4-(2-{7-[(methoxyimino)(phenyl)methyl]-3-oxo-2,3-dihydro-4*H*-1,4-benzothiazin-4-yl}ethoxy)phenyl]-2-(2,2,2-trifluoroethoxy)-propanoate, ethyl 2-ethoxy-3-[4-(2-{6-[(methoxyimino)(phenyl)methyl]-3-oxo-2,3-dihydro-4*H*-1,4-benzoxazin-4-yl}ethoxy)phenyl]propanoate, ethyl 2-ethoxy-3-[4-(2-{7-[(hydroxyimino)(phenyl)methyl]-3-oxo-2,3-dihydro-4*H*-1,4-benzothiazin-4-yl}-ethoxy)phenyl]propanoate, ethyl 3-[4-(2-{7-[(hydroxyimino)(phenyl)methyl]-3-oxo-2,3-dihydro-4*H*-1,4-benzothiazin-4-yl}ethoxy)phenyl]-2-(2,2,2-trifluoroethoxy)-propanoate, ethyl 2-ethoxy-3-[4-(2-{6-[(hydroxyimino)(phenyl)methyl]-3-oxo-2,3-dihydro-4*H*-1,4-benzoxazin-4-yl}ethoxy)phenyl]propanoate, 2-ethoxy-3-[4-(2-{7-[(hydroxyimino)(phenyl)methyl]-3-oxo-2,3-dihydro-4*H*-1,4-benzothiazin-4-yl}-ethoxy)phenyl]propanoic acid, 2-ethoxy-3-[4-(2-{7-[(methoxyimino)(phenyl)methyl]-3-oxo-2,3-dihydro-4*H*-1,4-benzothiazin-4-yl}ethoxy)phenyl]propanoic acid, 3-[4-(2-{7-[(hydroxyimino)(phenyl)methyl]-3-oxo-2,3-dihydro-4*H*-1,4-benzothiazin-4-yl}-ethoxy)phenyl]-2-(2,2,2-trifluoroethoxy)propanoic acid, 3-[4-(2-{7-[(methoxyimino)-(phenyl)methyl]-3-oxo-2,3-dihydro-4*H*-1,4-benzothiazin-4-yl}ethoxy)phenyl]-2-(2,2,2-trifluoroethoxy)propanoic acid, 2-ethoxy-3-[4-(2-{6-[(hydroxyimino)(phenyl)-methyl]-3-oxo-2,3-dihydro-4*H*-1, 4-benzoxazin-4-yl}ethoxy)phenyl]propanoic acid, 2-ethoxy-3-[4-(2-{6-[(methoxyimino)(phenyl)methyl]-3-oxo-2,3-dihydro-4*H*-1,4-benzoxazin-4-yl}ethoxy)phenyl]propanoic acid, enantiomers and diastereoisomers thereof and also pharmaceutically acceptable addition salts thereof with an acid or a base.

11. Process for the preparation of the compounds of formula (I) according to claim 1, **characterised in that** there is used as starting material a compound of (III) : 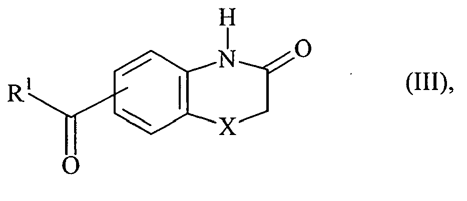 wherein R¹ and X are as defined for formula (I),
which is condensed in basic medium with a compound of formula (IV) : 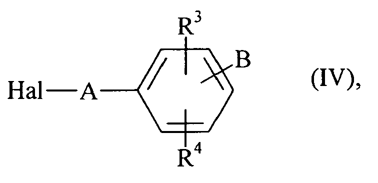 wherein A, B, R³ and R⁴ are as defined for formula (I) and Hal represents a halogen atom, to yield a compound of formula (V) : 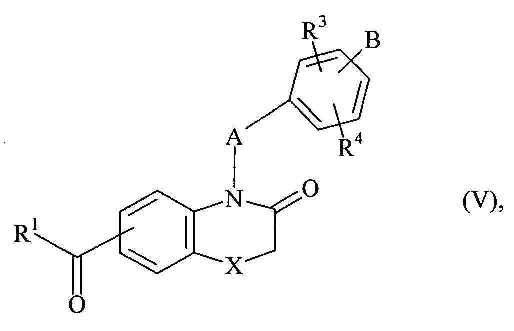 wherein R¹, R³, R⁴, A, B and X are as defined for formula (I),
which is subjected to the action of a compound of formula R²O-NH₂ wherein R² is as defined for formula (I) to yield a compound of formula (I) : 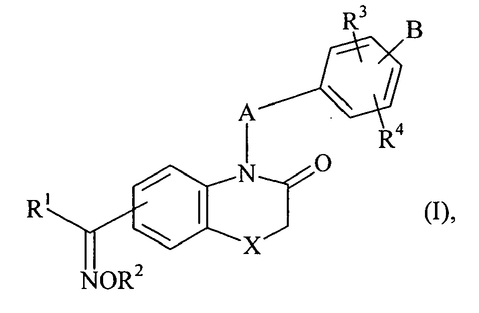 which may be purified according to a conventional separation technique, is converted, if desired, into addition salts with a pharmaceutically acceptable acid or base, and is optionally separated into isomers according to a conventional separation technique.

12. Process for the preparation of the compounds of formula (I) according to claim 1, **characterised in that** there is used as starting material a compound of (III) : 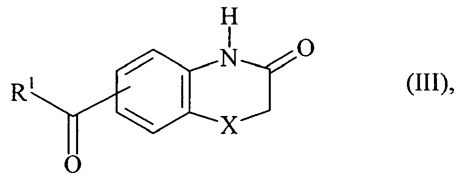 wherein R¹ and X are as defined for formula (I),
which is condensed with a compound of formula R²O-NH₂ wherein R² is as defined for formula (I) to yield a compound of formula (VI) : 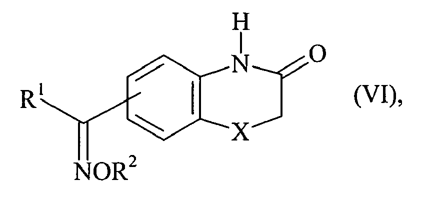 wherein R¹, R² and X are as defined for formula (I),
which is condensed in basic medium with a compound of formula (IV) : 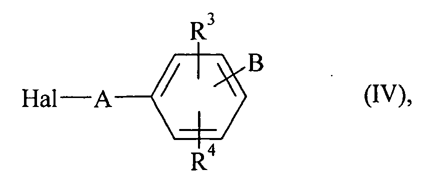 wherein A, B, R³ and R⁴ are as defined for formula (I) and Hal represents a halogen atom, to yield a compound of formula (I) : 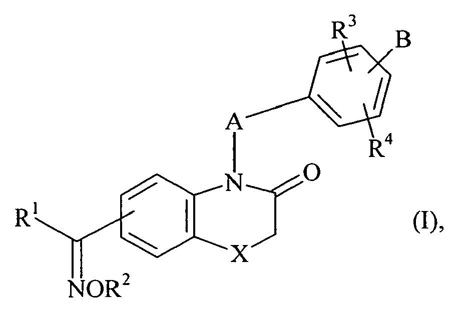 which may be purified according to a conventional separation technique, is converted, if desired, into addition salts with a pharmaceutically acceptable acid or base, and is optionally separated into isomers according to a conventional separation technique.

13. Pharmaceutical compositions comprising as active ingredient at least one compound of formula (I) according to any one of claims 1 to 10, or a pharmaceutically acceptable addition salt thereof with an acid or a base, alone or in combination with one or more pharmaceutically acceptable excipients.

14. Pharmaceutical compositions according to claim 13 for use in the manufacture of a medicament for the treatment and/or prophylaxis of hyperglycaemia, dyslipidaemia and, more especially, in the treatment of non-insulin-dependent type II diabetes, insulin resistance, glucose intolerance, disorders associated with syndrome X, coronary artery disease and other cardiovascular diseases, renal disorders, retinopathy, disorders associated with the activation of endothelial cells, psoriasis, polycystic ovary syndrome, dementia, osteoporosis, intestinal inflammatory disorders, myotonic dystrophy, pancreatitis, arteriosclerosis, xanthoma, and also in the treatment or the prevention of type I diabetes, obesity, the regulation of appetite, anorexia, bulimia, anorexia nervosa, as well as cancer pathologies and especially hormone-dependent

15. Association comprising a compound of formula (I) according to any one of claims 1 to 10 and an antioxidant agent.

16. Association according to claim 15, wherein the compound of formula (I) is 3-{4-[2-(6-(hydroxyiminophenyl)methyl-3-oxo-2,3-dihydro-4*H*-benzo[1,4]oxazin-4-yl)ethoxy] phenyl}-2-ethoxypropanoic acid, enantiomers and diastereoisomers thereof and also pharmaceutically acceptable addition salts thereof with an acid or a base.

17. Association according to claim 15 or 16, wherein the antioxidant agent is coenzyme Q₁₀.

18. Association according to claim 15 or 16, wherein the antioxidant agent is vitamin E.

19. Association according to claim 15 or 16 which is 3-{4-[2-(6-(hydroxyiminophenyl)-methyl-3-oxo-2,3-dihydro-4*H*-benzo[1,4]oxazin-4-yl)ethoxy]phenyl}-2-ethoxy propanoic acid and coenzyme Q₁₀.

20. Pharmaceutical compositions comprising as active ingredient an association according to any one of claims 15 to 19, alone or in combination with one or more pharmaceutically acceptable excipients..

21. Pharmaceutical compositions according to claim 20 for use in the manufacture of a medicament for the treatment and/or prevention of obesity.

22. Pharmaceutical compositions according to claim 20 for use in the manufacture of a medicament for the treatment and/or prevention of overweight **characterised by** a body mass index greater than 25 and less than 30.

23. Use of an association according to any one of claims 15 to 19 in obtaining pharmaceutical compositions intended for the treatment and/or prevention of obesity.

24. Use of an association according to any one of claims 15 to 19 in obtaining pharmaceutical compositions intended for the treatment and/or prevention of obesity caused by a therapeutic treatment.

25. Use of an association according to any one of claims 15 to 19 in obtaining pharmaceutical compositions intended for the treatment and/or prevention of obesity caused by a treatment of type I or type II diabetes.

26. Use of an association according to any one of claims 15 to 19 in obtaining pharmaceutical compositions intended for the treatment and/or prevention of overweight **characterised by** a body mass index greater than 25 and less than 30.

27. Use of an association according to any one of claims 15 to 19 in obtaining pharmaceutical compositions intended for the treatment and/or prevention of overweight **characterised by** a body mass index greater than 25 and less than 30 caused by a therapeutic treatment.

28. Use of an association according to any one of claims 15 to 19 in obtaining pharmaceutical compositions intended for the treatment and/or prevention of overweight **characterised by** a body mass index greater than 25 and less than 30 caused by a treatment of type I or type II diabetes.

## Patentansprüche

1. Verbindungen der Formel (I): 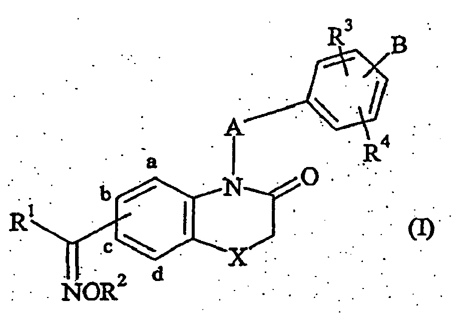 in der:
• X ein Sauerstoff- oder Schwefelatom bedeutet,
• A eine (C₁-C₆)-Alkylenkette darstellt, bei der eine CH₂-Gruppe gegebenenfalls durch ein Heteroatom ausgewählt aus Sauerstoff oder Schwefel oder durch eine Gruppe NRₐ (worin Rₐ ein Wasserstoffatom oder eine geradkettige oder verzweigte (C₁-C₆)-Alkylgruppe darstellt), oder durch eine Phenylen- oder Naphthylengruppe ersetzt ist,
• R¹ und R², die gleichartig oder verschieden sind, ein Wasserstoffatom oder eine geradkettige oder verzweigte (C₁-C₆)-Alkyl-, geradkettige oder verzweigte (C₂-C₆)-Alkenyl-, geradkettige oder verzweigte (C₂-C₆)-Alkinyl-, Aryl-, geradkettige oder verzweigte Aryl-(C₁-C₆)-alkyl-, geradkettige oder verzweigte Aryl-(C₂-C₆)-alkenyl-, geradkettige oder verzweigte Aryl-(C₂-C₆)-alkinyl-, Heteroaryl-, geradkettige oder verzweigte Heteroaryl-(C₁-C₆)-alkyl-, geradkettige oder verzweigte Heteroaryl-(C₂-C₆)-alkenyl-, geradkettige oder verzweigte Heteroaryl-(C₂-C₆)-alkinyl-, (C₃-C₈)-Cycloalkyl-, geradkettige oder verzweigte (C₃-C₈)-Cycloalkyl-(C₁-C₆)-alkyl- oder geradkettige oder verzweigte Polyhalogen-(C₁-C₆)-alkyl-Gruppe bedeuten,
• R³ und R⁴, die gleichartig oder verschieden sind, ein Wasserstoffatom oder Halogenatom oder eine Gruppe R, OR oder NRR' bedeuten, worin R und R', die gleichartig oder verschieden sind, ein Wasserstoffatom oder eine geradkettige oder verzweigte (C₁-C₆)-Alkyl-, geradkettige oder verzweigte (C₂-C₆)-Alkenyl-, geradkettige oder verzweigte (C₂-C₆)-Alkinyl-, Aryl-, geradkettige oder verzweigte Aryl-(C₁-C₆)-alkyl-, geradkettige oder verzweigte Aryl-(C₂-C₆)-alkenyl-, geradkettige oder verzweigte Aryl-(C₂-C₆)-alkinyl-, Heteroaryl-, geradkettige oder verzweigte Heteroaryl-(C₁-C₆)-alkyl-, geradkettige oder verzweigte Heteroaryl-(C₂-C₆)-alkenyl-, geradkettige oder verzweigte Heteroaryl-(C₂-C₆)-alkinyl-, (C₃-C₈)-Cycloalkyl-, geradkettige oder verzweigte (C₃-C₈)-Cycloalkyl-(C₁-C₆)-alkyl- oder geradkettige oder verzweigte Polyhalogen-(C₁-C₆)-alkyl-Gruppe bedeuten, oder R³ und R⁴ gemeinsam mit den sie tragenden Kohlenstoffatomen, wenn sie von zwei benachbarten Kohlenstoffatomen getragen werden, einen Ring mit 5 oder 6 Kettengliedern bilden, der gegebenenfalls ein Heteroatom ausgewählt aus Sauerstoff, Schwefel und Stickstoff enthalten kann,
• B eine geradkettige oder verzweigte (C₁-C₆)-Alkyl- oder geradkettige oder verzweigte (C₂-C₆)-Alkenylgruppe bedeutet, wobei diese Gruppen substituiert sind durch:
◆ eine Gruppe der Formel (II): 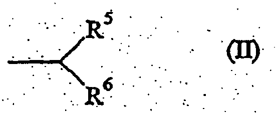 in der:
* R⁵ eine Gruppe 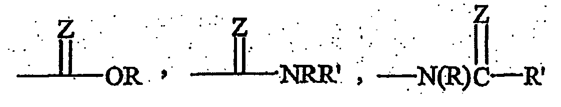 oder 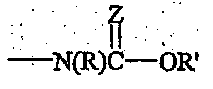 darstellt, worin Z ein Sauerstoff- oder Schwefelatom darstellt und R und R', die gleichartig oder verschieden sind, die oben angegebenen Bedeutungen besitzen,
* und R⁶ eine Arylgruppe, eine Arylalkylgruppe, deren Alkylrest 1 bis 6 Kohlenstoffatome enthält und geradkettig oder verzweigt sein kann, Heteroarylgruppe, Heteroarylalkylgruppe, deren Alkylrest 1 bis 6 Kohlenstoffatome enthält und geradkettig oder verzweigt sein kann, CN, Tetrazol, -OR, -NRR¹, 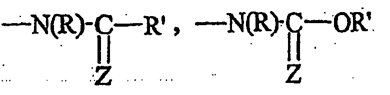 oder 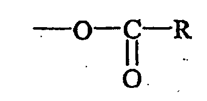 darstellt, worin Z die oben angegebenen Bedeutungen besitzt und R und R', die gleichartig oder verschieden sein können, die gleichen Bedeutungen besitzen wie oben angegeben,
◆ oder durch eine Gruppe R⁷, worin R⁷ eine Gruppe CN, Tetrazol, 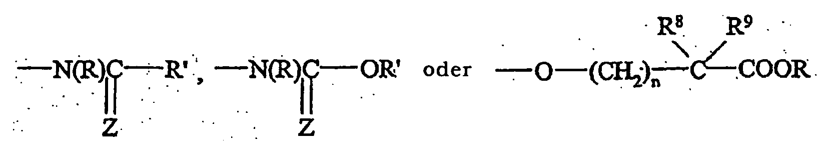 darstellt, worin Z die oben angegebenen Bedeutungen besitzt und R und R', die gleichartig oder verschieden sind, die oben angegebenen Bedeutungen besitzen, n 0, 1, 2, 3, 4, 5 oder 6 bedeutet und R⁸ und R⁹, die gleichartig oder verschieden sind, ein Wasserstoffatom oder eine geradkettige oder verzweigte (C₁-C₆)-Alkylgruppe bedeuten, mit der Maßgabe, dass R⁸ und R⁹ nicht gleichzeitig Wasserstoffatome bedeuten,
oder B eine Gruppe der Formel (II) oder eine Gruppe R⁷ bedeutet, wie sie oben definiert worden sind,
mit der Maßgabe, dass:
* das Oxim R¹-C(=N-OR²)- in der Konfiguration Z oder E vorliegen kann,
* man unter Aryl eine Phenyl-, Naphthyl- oder Biphenylgruppe versteht, wobei diese Gruppen gegebenenfalls teilweise hydriert sein können,
* man unter Heteroaryl eine aromatische mono- oder bicyclische Gruppe versteht, die 5 bis 10 Kohlenstoffatome aufweist und die gegebenenfalls teilweise an einem der Ringe im Fall von bicyclischen Heteroarylgruppen hydriert ist, und 1 bis 3 Heteroatome ausgewählt aus Sauerstoff, Stickstoff und Schwefel enthält,
wobei die in dieser Weise definierten Aryl- und Heteroarylgruppen gegebenenfalls durch 1 bis 3 gleichartige oder verschiedenartige Gruppen sind, ausgewählt aus geradkettigem oder verzweigtem (C₁-C₆)-Alkyl, geradkettigem oder verzweigtem (C₁-C₆)-Polyhalogenalkyl, geradkettigem oder verzweigtem (C₁-C₆)-Alkoxy, Hydroxy, Carb-oxy, Formyl, geradkettigem oder verzweigtem (C₁-C₆)-Acyl, Aroyl, NR_{b}R_{c} (worin R_{b} und R_{c}, die gleichartig oder verschieden sind, ein Wasserstoffatom, eine geradkettige oder verzweigte (C₁-C₆)-Alkylgruppe, Arylgruppe oder Heteroarylgruppe bedeuten), Ester, Amido, Nitro, Cyano oder Halogenatomen,
deren Enantiomere und Diastereoisomere sowie deren Additionssalze mit einer pharmazeutisch annehmbaren Säure oder Base.

2. Verbindungen der Formel (I) nach Anspruch 1, worin die Gruppe R¹-C(=N-OR²)- in der b- oder c-Stellung steht, deren Enantiomere und Diastereoisomere sowie deren Additionssalze mit einer pharmazeutisch annehmbaren Säure oder Base.

3. Verbindungen der Formel (I) nach Anspruch 1, worin R³ und R⁴ ein Wasserstoffatom bedeuten, deren Enantiomere und Diastereoisomere sowie deren Additionssalze mit einer pharmazeutisch annehmbaren Säure oder Base.

4. Verbindungen der Formel (I) nach Anspruch 1, worin A eine Ethylenoxygruppe bedeutet, deren Enantiomere und Diastereoisomere sowie deren Additionssalze mit einer pharmazeutisch annehmbaren Säure oder Base.

5. Verbindungen der Formel (I) nach Anspruch 1, worin R² ein Wasserstoffatom bedeutet, deren Enantiomere und Diastereoisomere sowie deren Additionssalze mit einer pharmazeutisch annehmbaren Säure oder Base.

6. Verbindungen der Formel (I) nach Anspruch 1, worin R² eine Alkylgruppe bedeutet, deren Enantiomere und Diastereoisomere sowie deren Additionssalze mit einer pharmazeutisch annehmbaren Säure oder Base.

7. Verbindungen der Formel (I) nach Anspruch 1, worin R¹ eine nichtsubstituierte oder substituierte Phenylgruppe bedeutet, deren Enantiomere und Diastereoisomere sowie deren Additionssalze mit einer pharmazeutisch annehmbaren Säure oder Base.

8. Verbindungen der Formel (I) nach Anspruch 1, worin B eine Alkylgruppe bedeutet, die durch eine Gruppe 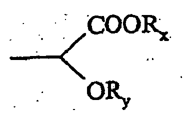 substituiert ist, in der Rₓ und R_{y}, die gleichartig oder verschieden sind, ein Wasserstoffatom oder eine Alkylgruppe bedeuten, deren Enantiomere und Diastereoisomere sowie deren Additionssalze mit einer pharmazeutisch annehmbaren Säure oder Base.

9. Verbindungen der Formel (I) nach Anspruch 1, worin B eine Gruppe 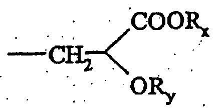 darstellt, in der Rₓ und R_{y}, die gleichartig oder verschieden sind, ein Wasserstoffatom oder eine Alkylgruppe bedeuten, deren Enantiomere und Diastereoisomere sowie deren Additionssalze mit einer pharmazeutisch annehmbaren Säure oder Base.

10. Verbindungen der Formel (I) nach Anspruch 1, nämlich 2-Ethoxy-3-[4-(2-{7-[(methoxyimino)(phenyl)-methyl]-3-oxo-2,3-dihydro-4H-1,4-benzothiazin-4-yl}-ethoxy)-phenyl]-propansäureethylester, 3-[4-(2-{7-[(Methoxyimino)(phenyl)-methyl]-3-oxo-2,3-dihydro-4H-1,4-benzothiazin-4-yl}-ethoxy)-phenyl]-2-(2,2,2-trifluorethoxy)-propansäureethylester, 2-Ethoxy-3-[4-(2-{6-[(methoxyimino)(phenyl)-methyl]-3-oxo-2,3-dihydro-4*H*-1,4-benzoxazin-4-yl}-ethoxy)-phenyl]-propansäureethylester, 2-Ethoxy-3-[4-(2-{7-[(hydroxyimino)(phenyl)-methyl-3-oxo-2,3-dihydro-4*H-*1,4-benzothiazin-4-yl}-ethoxy)-phenyl]-propansäureethyester, 3-[4-(2-{7-[(Hydroxyimino)(phenyl)-methyl]-3-oxo-2,3-dihydro-4*H*-1,4-benzothiazin-4-yl}-ethoxy)-phenyl]-2-(2,2,2-trifluorethoxy)-propansäureethylester, 2-Ethoxy-3-[4-(2-{6-[(hydroxyimino)(phenyl)-methyl]-3-oxo-2,3-dihydro-4*H*-1,4-benzoxazin-4-yl}-ethoxy)-phenyl]-propansäureethylester, 2-Ethoxy-3-[4-(2-{7-[(hydroxyimino)(phenyl)-methyl]-3-oxo-2,3-dihydro-4*H*-1,4-benzothiazin-4-yl}-ethoxy)-phenyl]-propansäure, 2-Ethoxy-3-[4-(2-{7-[(methoxyimino)(phenyl)-methyl]-3-oxo-2,3-dihydro-4*H*-1,4-benzothiazin-4-yl}-ethoxy)-phenyl]-propansäure, 3-[4-(2-{7-[(Hydroxyimino)(phenyl)-methyl]-3-oxo-2,3-dihydro-4*H*-1,4-benzothiazin-4-yl}-ethoxy)-phenyl]-2-(2,2,2-trifluorethoxy)-propansäure, 3-[4-(2-{7-[(Methoxyimino)(phenyl)-methyl]-3-oxo-2,3-dihydro-4*H*-1,4-benzothiazin-4-yl}-ethoxy)-phenyl]-2-(2,2,2-trifluorethoxy)-propansäure, 2-Ethoxy-3-[4-(2-{6-[(hydroxyimino)(phenyl)-methyl]-3-oxo-2,3-dihydro-4H-1,4-benzoxazin-4-yl}-ethoxy)-phenyl]-propansäure, 2-Ethoxy-3-[4-(2-{6-[(methoxyimino)(phenyl)-methyl]-3-oxo-2,3-dihydro-4*H*-1,4-benzoxazin-4-yl}-ethoxy)-phe-nyl]-propansäure, deren Enantiomere und Diastereoisomere sowie deren Additionssalze mit einer pharmazeutisch annehmbaren Säure oder Base.

11. Verfahren zur Herstellung der Verbindungen der Formel (I) nach Anspruch 1, **dadurch gekennzeichnet, dass** man als Ausgangsprodukt die Verbindung der Formel (III) verwendet: 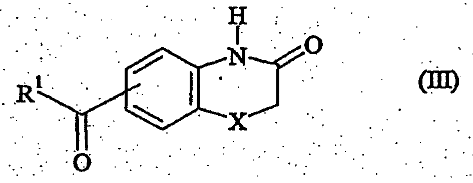 in der R¹ und X die bezüglich der Formel (I) angegebenen Bedeutungen besitzen, welche man in basischem Medium mit einer Verbindung der Formel (IV) kondensiert: 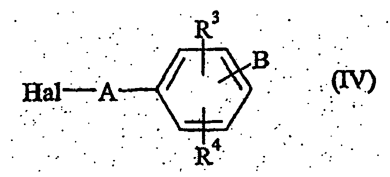 in der A, B, R³ und R⁴ die bezüglich der Formel (I) angegebenen Bedeutungen besitzen und Hal ein Halogenatom bedeutet, zur Bildung der Verbindung der Formel (V): 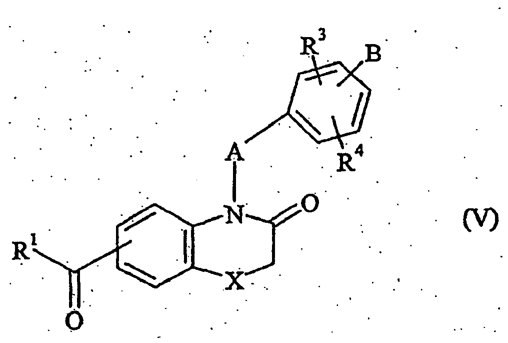 in der R¹, R³, R⁴, A, B und X die bezüglich der Formel (I) angegebenen Bedeutungen besitzen,
welche man der Einwirkung einer Verbindung der Formel R²O-NH₂, worin R² die bezüglich der Formel (I) angegebenen Bedeutungen besitzt, unterwirft zur Bildung der Verbindung der Formel (I): 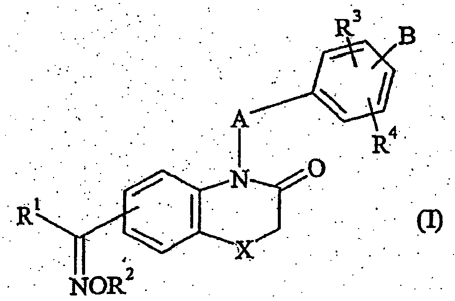 welche mit Hilfe einer klassischen Trennmethode gereinigt werden kann, welche man gewünschtenfalls mit einer pharmazeutisch annehmbaren Säure oder Base in ihre Additionssalze überführt und die man gegebenenfalls mit Hilfe einer klassischen Trennmethode in ihre Isomeren auftrennt.

12. Verfahren zur Herstellung der Verbindungen der Formel (I) nach Anspruch 1, **dadurch gekennzeichnet, dass** man als Ausgangsprodukt eine Verbindung der Formel (III) verwendet: 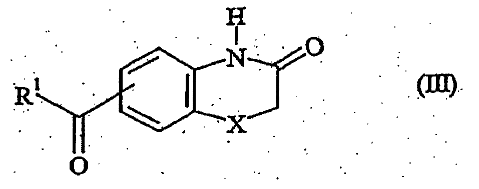 in der R¹ und X die bezüglich der Formel (I) angegebenen Bedeutungen besitzen, welche man mit einer Verbindung der Formel R²O-NH₂, worin R² die bezüglich der Formel (I) angegebenen Bedeutungen besitzt, kondensiert zur Bildung der Verbindung der Formel (VI): 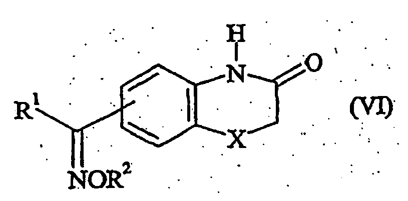 in der R¹, R² und X die bezüglich der Formel (I) angegebenen Bedeutungen besitzen,
welche man in basischem Medium mit einer Verbindung der Formel (IV) kondensiert: 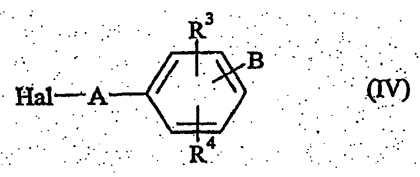 in der A, B, R³ und R⁴ die bezüglich der Formel (I) angegebenen Bedeutungen besitzen und Hal ein Halogenatom bedeutet, zur Bildung der Verbindung der Formel (I): 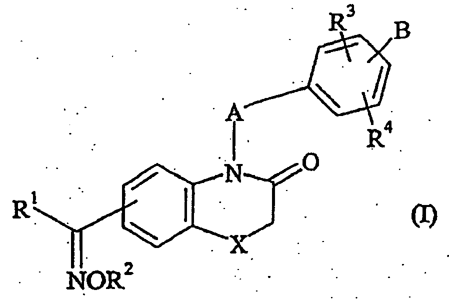 welche mit Hilfe einer klassischen Trennmethode gereinigt werden kann, welche man gewünschtenfalls mit einer pharmazeutisch annehmbaren Säure oder Base in ihre Additionssalze überführt und die man gegebenenfalls mit Hilfe einer klassischen Trennmethode in ihre Isomeren auftrennt.

13. Pharmazeutische Zubereitungen enthaltend als Wirkstoff mindestens eine Verbindung der Formel (I) nach einem der Ansprüche 1 bis 10 oder eines ihrer Additionssalze mit einer pharmazeutisch annehmbaren Säure oder Base, alleine oder in Kombination mit einem oder mehreren pharmazeutisch annehmbaren Hilfsstoffen.

14. Pharmazeutische Zubereitungen nach Anspruch 13 nützlich für die Herstellung eines Arzneimittels zur Behandlung und/oder Prophylaxe von Hyperglykämie, Dyslipidämie, insbesondere zur Behandlung des nicht insulinabhängigen Diabetes Typ II, der Insulinresistenz, der Glucoseunverträglichkeit, von Störungen, die mit dem Syndrom X verknüpft sind, Erkrankungen der Koronararterien und anderen kardiovaskulären Erkrankungen, Nierenerkrankungen, Retinopathien, Störungen, die mit einer Aktivierung der endothelialen Zellen verknüpft sind, Psoriasis, des polycystischen Ovarialsyndroms, der Demenz, der Osteoporose, Magen-Darm-Entzündungserkrankungen, myotonischen Dystrophien, Pankreatitis, Arteriosklerose, Xanthom, jedoch auch zur Behandlung oder zur Vorbeugung von Diabetes Typ I, der Fettsucht, der Appetitsteuerung, von Anorexie, Bulimie, nervöser Anorexie sowie von Krebserkrankungen und insbesondere hormonabhängigen Krebsen, wie Brustkrebs und Darmkrebs und als Inhibitoren der Angiogenese.

15. Kombination enthaltend eine Verbindung der Formel (I) nach einem der Ansprüche 1 bis 10 und ein antioxidierendes Mittel.

16. Kombination nach Anspruch 15, worin die Verbindung der Formel (I) 3-{4-[2-(6-(Hydroxyimino-phenyl)-methyl-3-oxo-2,3-dihydro-4*H*-benzo[1,4]oxazin-4-yl)-ethoxy]-phenyl}-2-ethoxy-propansäure, ihre Enantiomere und Diastereoisomere sowie ihre Additionssalze mit einer pharmazeutisch annehmbaren Säure oder Base ist.

17. Kombination nach Anspruch 15 oder 16, worin das Antioxidationsmittel Coenzym Q₁₀ ist.

18. Kombination nach Anspruch 15 oder 16, worin das Antioxidationsmittel Vitamin E ist.

19. Kombination nach Anspruch 15 oder 16, welche 3-{4-[2-(6-(Hydroxyimino-phenyl)-methyl-3-oxo-2,3-dihydro-4*H*-benzo[1,4]oxazin-4-yl)-ethoxy]-phenyl}-2-ethoxypropan-säure und Coenzym Q₁₀ umfasst.

20. Pharmazeutische Zubereitungen enthaltend als Wirkstoff eine Kombination nach einem der Ansprüche 15 bis 19 allein oder in Kombination mit einem oder mehreren pharmazeutisch annehmbaren Hilfsstoffen.

21. Pharmazeutische Zubereitungen nach Anspruch 20, die nützlich sind für die Herstellung eines Arzneimittels zur Behandlung und/oder Vorbeugung der Fettsucht.

22. Pharmazeutische Zubereitungen nach Anspruch 20, die nützlich sind für Übergewicht, **gekennzeichnet durch** einen Körpergewichtsindex größer als 25 und weniger als 30.

23. Verwendung einer Kombination nach einem der Ansprüche 15 bis 19 für die Herstellung von pharmazeutischen Zubereitungen, die bestimmt sind zur Behandlung und/oder Vorbeugung der Fettsucht.

24. Verwendung einer Kombination nach einem der Ansprüche 15 bis 19 zur Herstellung von pharmazeutischen Zubereitungen, die bestimmt sind zur Behandlung und/oder der Vorbeugung der Fettsucht, die durch eine therapeutische Behandlung verursacht ist.

25. Verwendung einer Kombination nach einem der Ansprüche 15 bis 19 zur Herstellung von pharmazeutischen Zubereitungen, die bestimmt sind zur Behandlung und/oder zur Vorbeugung der Fettsucht, die durch die Behandlung von Diabetes Typ I oder II verursacht ist.

26. Verwendung einer Kombination nach einem der Ansprüche 15 bis 19 für die Herstellung von pharmazeutischen Zubereitungen, die bestimmt sind zur Behandlung und/oder zur Vorbeugung von Übergewicht, **gekennzeichnet durch** einen Körpergewichtsindex größer als 25 und weniger als 30.

27. Verwendung einer Kombination nach einem der Ansprüche 15 bis 19 für die Herstellung von pharmazeutischen Zubereitungen, die bestimmt sind zur Behandlung und/oder Vorbeugung von Übergewicht, **gekennzeichnet durch** einen Körpergewichtsindex größer als 25 und weniger als 30, welches **durch** eine therapeutische Behandlung verursacht ist.

28. Verwendung einer Kombination nach einem der Ansprüche 15 bis 19 für die Herstellung von pharmazeutischen Zubereitungen, die bestimmt sind zur Behandlung und/oder zur Vorbeugung von Übergewicht, **gekennzeichnet durch** einen Körpergewichtsindex größer als 25 und weniger als 30, die verursacht sind durch eine Behandlung des Diabetes Typ I oder II.
